# EUROPEAN PATENT APPLICATION

(11) **EP 4 617 366 A1**
(43) Date of publication of application: **17.09.2025**
(21) Application number: 23888704.6
(22) Date of filing: 07.11.2023
(51) Int. Cl.: C12N 15/13, C07K 16/18, C12N 1/15, C12N 1/19, C12N 1/21, C12N 5/10, C12N 15/63, G01N 33/53

(54) **ANTIBODY AGAINST COATOMER PROTEIN COMPLEX SUBUNIT BETA 2**

(30) Priority: 07.11.2022 JP 2022178493
(71) Applicant: ISM Co., Ltd., Tokyo 1020082 (JP)
(72) Inventor: MIYATO, Mitsuru, Tokyo 102-0082 (JP); MATSUZAKI, Juntaro, Tokyo 105-8512 (JP)
(74) Representative: D Young & Co LLP
(86) International application number: PCT/JP2023/040066
(87) International publication number: WO 2024/101357

(57) **Abstract**

Provided is an antibody against coatomer protein complex subunit beta 2 (CO PB2) or an antigen binding fragment thereof, comprising any of the following CDR sets (a) to (c): (a) heavy chain CDR1 consisting of the amino acid sequence represented by SEQ ID NO: 1, heavy chain CDR2 consisting of the amino acid sequence represented by SEQ ID NO: 2, heavy chain CDR3 consisting of the amino acid sequence represented by SEQ ID NO: 3, light chain CDR1 consisting of the amino acid sequence represented by SEQ ID NO: 4, light chain CDR2 consisting of the amino acid sequence represented by SEQ ID NO: 5, and light chain CDR3 consisting of the amino acid sequence represented by SEQ ID NO: 6, (b) heavy chain CDR1 consisting of the amino acid sequence represented by SEQ ID NO: 7, heavy chain CDR2 consisting of the amino acid sequence represented by SEQ ID NO: 8, heavy chain CDR3 consisting of the amino acid sequence represented by SEQ ID NO: 9, light chain CDR1 consisting of the amino acid sequence represented by SEQ ID NO: 10, light chain CDR2 consisting of the amino acid sequence represented by SEQ ID NO: 11, and light chain CDR3 consisting of the amino acid sequence represented by SEQ ID NO: 12, (c) heavy chain CDR1 consisting of the amino acid sequence represented by SEQ ID NO: 13, heavy chain CDR2 consisting of the amino acid sequence represented by SEQ ID NO: 14, heavy chain CDR3 consisting of the amino acid sequence represented by SEQ ID NO: 15, light chain CDR1 consisting of the amino acid sequence represented by SEQ ID NO: 16, light chain CDR2 consisting of the amino acid sequence represented by SEQ ID NO: 17, and light chain CDR3 consisting of the amino acid sequence represented by SEQ ID NO: 18.

## Description

### Technical Field

The present invention relates to an antibody against COPB2 capable of detecting coatomer protein complex subunit beta 2 (COPB2), or the like.

### Background Art

In recent years, severe acute respiratory syndrome coronavirus 2 (SARS-CoV-2) infection (COVID-19) has rampaged throughout the world.

Persons infected with COVID-19 manifest cold-like initial symptoms such as fever or cough after a relatively long incubation period of 1 to 14 days. However, most of the infected persons have mild symptoms or asymptomatic infections. A problem of COVID-19 is that the remaining fraction of the infected persons develops a severe respiratory problem such as acute respiratory distress syndrome (ARDS), which is further complicated by myocarditis, vasculitis, encephalomeningitis, or the like, falling into a life-threatening situation, i.e., worsening.

SARS-CoV-2, a causative virus of COVID-19, is a virus belonging to the genus *Coronavirus*, and PCR or the like is used in its detection. However, such testing methods merely determine the presence or absence of infection and, unfortunately, cannot predict subsequent worsening.

Patent Literature 1 discloses a method for determining the possibility of worsening in a COVID-19 patient, comprising measuring levels of one or more marker proteins present in exosomes derived from the patient, wherein COPB2 is one of the marker proteins.

COPB2 is one of the non-clathrin-coated vesicular subunits constituting coatomer which is involved in the membrane transport between the endoplasmic reticulum and the Golgi apparatus, and is a coatomer protein involved in the membrane transport between the endoplasmic reticulum and the Golgi apparatus. COPB2, as the coatomer protein, plays an important role in embryonic development or tumor progression, participates in various pathological conditions, and also participates in the control of cancer cell proliferation, metastasis, or the like, and as such, is under research as an important factor for many cancers (Non-Patent Literature 1).

### Citation List

### Patent Literature

Patent Literature 1: International Publication No. WO 2022/071601

### Non-Patent Literature

Non-Patent Literature 1: Clin Transl Oncol, 2021 (11): 2195-2205

### Summary of Invention

### Technical Problem

An object of the present invention is to provide a novel antibody capable of detecting COPB2 protein, or the like.

### Solution to Problem

The present inventors have conducted diligent studies and consequently found a novel antibody having CDRs consisting of particular sequences.

Specifically, the present invention is as follows.
(1) An antibody against coatomer protein complex subunit beta 2 (COPB2) or an antigen binding fragment thereof, comprising any of the following CDR sets (a) to (c):
   (a)
      heavy chain CDR1 consisting of the amino acid sequence represented by SEQ ID NO: 1,
      heavy chain CDR2 consisting of the amino acid sequence represented by SEQ ID NO: 2,
      heavy chain CDR3 consisting of the amino acid sequence represented by SEQ ID NO: 3,
      light chain CDR1 consisting of the amino acid sequence represented by SEQ ID NO: 4,
      light chain CDR2 consisting of the amino acid sequence represented by SEQ ID NO: 5, and
      light chain CDR3 consisting of the amino acid sequence represented by SEQ ID NO: 6,
   (b)
      heavy chain CDR1 consisting of the amino acid sequence represented by SEQ ID NO: 7,
      heavy chain CDR2 consisting of the amino acid sequence represented by SEQ ID NO: 8,
      heavy chain CDR3 consisting of the amino acid sequence represented by SEQ ID NO: 9,
      light chain CDR1 consisting of the amino acid sequence represented by SEQ ID NO: 10,
      light chain CDR2 consisting of the amino acid sequence represented by SEQ ID NO: 11, and
      light chain CDR3 consisting of the amino acid sequence represented by SEQ ID NO: 12,
   (c)
      heavy chain CDR1 consisting of the amino acid sequence represented by SEQ ID NO: 13,
      heavy chain CDR2 consisting of the amino acid sequence represented by SEQ ID NO: 14,
      heavy chain CDR3 consisting of the amino acid sequence represented by SEQ ID NO: 15,
      light chain CDR1 consisting of the amino acid sequence represented by SEQ ID NO: 16,
      light chain CDR2 consisting of the amino acid sequence represented by SEQ ID NO: 17, and
      light chain CDR3 consisting of the amino acid sequence represented by SEQ ID NO: 18.
(1-1) An antibody against coatomer protein complex subunit beta 2 (CO PB2) or an antigen binding fragment thereof, comprising any of the following CDR sets (a1) to (c1):
   (a1)
      heavy chain CDR1 consisting of the amino acid sequence represented by SEQ ID NO: 1 or an amino acid sequence differing by having addition, substitution, or deletion of one to several amino acids from the amino acid sequence represented by SEQ ID NO: 1,
      heavy chain CDR2 consisting of the amino acid sequence represented by SEQ ID NO: 2 or an amino acid sequence differing by having addition, substitution, or deletion of one to several amino acids from the amino acid sequence represented by SEQ ID NO: 2,
      heavy chain CDR3 consisting of the amino acid sequence represented by SEQ ID NO: 3 or an amino acid sequence differing by having addition, substitution, or deletion of one to several amino acids from the amino acid sequence represented by SEQ ID NO: 3,
      light chain CDR1 consisting of the amino acid sequence represented by SEQ ID NO: 4 or an amino acid sequence differing by having addition, substitution, or deletion of one to several amino acids from the amino acid sequence represented by SEQ ID NO: 4,
      light chain CDR2 consisting of the amino acid sequence represented by SEQ ID NO: 5 or an amino acid sequence differing by having addition, substitution, or deletion of one to several amino acids from the amino acid sequence represented by SEQ ID NO: 5, and
      light chain CDR3 consisting of the amino acid sequence represented by SEQ ID NO: 6 or an amino acid sequence differing by having addition, substitution, or deletion of one to several amino acids from the amino acid sequence represented by SEQ ID NO: 6,
   (b1)
      heavy chain CDR1 consisting of the amino acid sequence represented by SEQ ID NO: 7 or an amino acid sequence differing by having addition, substitution, or deletion of one to several amino acids from the amino acid sequence represented by SEQ ID NO: 7,
      heavy chain CDR2 consisting of the amino acid sequence represented by SEQ ID NO: 8 or an amino acid sequence differing by having addition, substitution, or deletion of one to several amino acids from the amino acid sequence represented by SEQ ID NO: 8,
      heavy chain CDR3 consisting of the amino acid sequence represented by SEQ ID NO: 9 or an amino acid sequence differing by having addition, substitution, or deletion of one to several amino acids from the amino acid sequence represented by SEQ ID NO: 9,
      light chain CDR1 consisting of the amino acid sequence represented by SEQ ID NO: 10 or an amino acid sequence differing by having addition, substitution, or deletion of one to several amino acids from the amino acid sequence represented by SEQ ID NO: 10,
      light chain CDR2 consisting of the amino acid sequence represented by SEQ ID NO: 11 or an amino acid sequence differing by having addition, substitution, or deletion of one to several amino acids from the amino acid sequence represented by SEQ ID NO: 11, and
      light chain CDR3 consisting of the amino acid sequence represented by SEQ ID NO: 12 or an amino acid sequence differing by having addition, substitution, or deletion of one to several amino acids from the amino acid sequence represented by SEQ ID NO: 12,
   (c1)
      heavy chain CDR1 consisting of the amino acid sequence represented by SEQ ID NO: 13 or an amino acid sequence differing by having addition, substitution, or deletion of one to several amino acids from the amino acid sequence represented by SEQ ID NO: 13,
      heavy chain CDR2 consisting of the amino acid sequence represented by SEQ ID NO: 14 or an amino acid sequence differing by having addition, substitution, or deletion of one to several amino acids from the amino acid sequence represented by SEQ ID NO: 14,
      heavy chain CDR3 consisting of the amino acid sequence represented by SEQ ID NO: 15 or an amino acid sequence differing by having addition, substitution, or deletion of one to several amino acids from the amino acid sequence represented by SEQ ID NO: 15,
      light chain CDR1 consisting of the amino acid sequence represented by SEQ ID NO: 16 or an amino acid sequence differing by having addition, substitution, or deletion of one to several amino acids from the amino acid sequence represented by SEQ ID NO: 16,
      light chain CDR2 consisting of the amino acid sequence represented by SEQ ID NO: 17 or an amino acid sequence differing by having addition, substitution, or deletion of one to several amino acids from the amino acid sequence represented by SEQ ID NO: 17, and
      light chain CDR3 consisting of the amino acid sequence represented by SEQ ID NO: 18 or an amino acid sequence differing by having addition, substitution, or deletion of one to several amino acids from the amino acid sequence represented by SEQ ID NO: 18.
(1-2) An antibody against coatomer protein complex subunit beta 2 (COPB2) or an antigen binding fragment thereof, comprising any of the following CDR sets (a2) to (c2):
   (a2)
      heavy chain CDR1 consisting of an amino acid sequence having 80% or higher identity to the amino acid sequence represented by SEQ ID NO: 1,
      heavy chain CDR2 consisting of an amino acid sequence having 80% or higher identity to the amino acid sequence represented by SEQ ID NO: 2,
      heavy chain CDR3 consisting of an amino acid sequence having 80% or higher identity to the amino acid sequence represented by SEQ ID NO: 3,
      light chain CDR1 consisting of an amino acid sequence having 80% or higher identity to the amino acid sequence represented by SEQ ID NO: 4,
      light chain CDR2 consisting of an amino acid sequence having 80% or higher identity to the amino acid sequence represented by SEQ ID NO: 5, and
      light chain CDR3 consisting of an amino acid sequence having 80% or higher identity to the amino acid sequence represented by SEQ ID NO: 6,
   (b2)
      heavy chain CDR1 consisting of an amino acid sequence having 80% or higher identity to the amino acid sequence represented by SEQ ID NO: 7,
      heavy chain CDR2 consisting of an amino acid sequence having 80% or higher identity to the amino acid sequence represented by SEQ ID NO: 8,
      heavy chain CDR3 consisting of an amino acid sequence having 80% or higher identity to the amino acid sequence represented by SEQ ID NO: 9,
      light chain CDR1 consisting of an amino acid sequence having 80% or higher identity to the amino acid sequence represented by SEQ ID NO: 10,
      light chain CDR2 consisting of an amino acid sequence having 80% or higher identity to the amino acid sequence represented by SEQ ID NO: 11, and
      light chain CDR3 consisting of an amino acid sequence having 80% or higher identity to the amino acid sequence represented by SEQ ID NO: 12,
   (c2)
      heavy chain CDR1 consisting of an amino acid sequence having 80% or higher identity to the amino acid sequence represented by SEQ ID NO: 13,
      heavy chain CDR2 consisting of an amino acid sequence having 80% or higher identity to the amino acid sequence represented by SEQ ID NO: 14,
      heavy chain CDR3 consisting of an amino acid sequence having 80% or higher identity to the amino acid sequence represented by SEQ ID NO: 15,
      light chain CDR1 consisting of an amino acid sequence having 80% or higher identity to the amino acid sequence represented by SEQ ID NO: 16,
      light chain CDR2 consisting of an amino acid sequence having 80% or higher identity to the amino acid sequence represented by SEQ ID NO: 17, and
      light chain CDR3 consisting of an amino acid sequence having 80% or higher identity to the amino acid sequence represented by SEQ ID NO: 18.
(2) An antibody against coatomer protein complex subunit beta 2 (COPB2) or an antigen binding fragment thereof, comprising any of the following heavy chain variable regions and/or light chain variable regions (d) to (f):
   (d)
      a heavy chain variable region comprising the amino acid sequence represented by SEQ ID NO: 19;
      a heavy chain variable region comprising an amino acid sequence differing by having addition, substitution, or deletion of one to several amino acids from the amino acid sequence represented by SEQ ID NO: 19; or
      a heavy chain variable region comprising an amino acid sequence having 80% or higher identity to the amino acid sequence represented by SEQ ID NO: 19, and/or
      a light chain variable region comprising the amino acid sequence represented by SEQ ID NO: 20;
      a light chain variable region comprising an amino acid sequence differing by having addition, substitution, or deletion of one to several amino acids from the amino acid sequence represented by SEQ ID NO: 20; or
      a light chain variable region comprising an amino acid sequence having 80% or higher identity to the amino acid sequence represented by SEQ ID NO: 20,
   (e)
      a heavy chain variable region comprising the amino acid sequence represented by SEQ ID NO: 21;
      a heavy chain variable region comprising an amino acid sequence differing by having addition, substitution, or deletion of one to several amino acids from the amino acid sequence represented by SEQ ID NO: 21; or
      a heavy chain variable region comprising an amino acid sequence having 80% or higher identity to the amino acid sequence represented by SEQ ID NO: 21, and/or
      a light chain variable region comprising the amino acid sequence represented by SEQ ID NO: 22;
      a light chain variable region comprising an amino acid sequence differing by having addition, substitution, or deletion of one to several amino acids from the amino acid sequence represented by SEQ ID NO: 22; or
      a light chain variable region comprising an amino acid sequence having 80% or higher identity to the amino acid sequence represented by SEQ ID NO: 22,
   (f)
      a heavy chain variable region comprising the amino acid sequence represented by SEQ ID NO: 23;
      a heavy chain variable region comprising an amino acid sequence differing by having addition, substitution, or deletion of one to several amino acids from the amino acid sequence represented by SEQ ID NO: 23; or
      a heavy chain variable region comprising an amino acid sequence having 80% or higher identity to the amino acid sequence represented by SEQ ID NO: 23, and/or
      a light chain variable region comprising the amino acid sequence represented by SEQ ID NO: 24;
      a light chain variable region comprising an amino acid sequence differing by having addition, substitution, or deletion of one to several amino acids from the amino acid sequence represented by SEQ ID NO: 24; or
      a light chain variable region comprising an amino acid sequence having 80% or higher identity to the amino acid sequence represented by SEQ ID NO: 24.

The antibody against coatomer protein complex subunit beta 2 (COPB2) or the antigen binding fragment thereof, comprising any of the heavy chain variable regions and/or the light chain variable regions (d) to (f) is preferably the antibody or the antigen binding fragment thereof according to any of (1) to (1-2).

Specifically, the antibody against coatomer protein complex subunit beta 2 (COPB2) or the antigen binding fragment thereof, comprising any of heavy chain variable regions and/or light chain variable regions (d) to (f), when being an antibody against coatomer protein complex subunit beta 2 (COPB2) or an antigen binding fragment thereof, comprising the heavy chain variable region and/or the light chain variable region (d), preferably comprises any one of the CDR set (a), (a1), or (a2) and more preferably comprises the CDR set (a); when being an antibody against coatomer protein complex subunit beta 2 (COPB2) or an antigen binding fragment thereof, comprising the heavy chain variable region and/or the light chain variable region (e), preferably comprises any one of the CDR set (b), (b1), or (b2) and more preferably comprises the CDR set (b); and when being an antibody against coatomer protein complex subunit beta 2 (COPB2) or an antigen binding fragment thereof, comprising the heavy chain variable region and/or the light chain variable region (f), preferably comprises any one of the CDR set (c), (c1), or (c2) and more preferably comprises the CDR set (c).

Such an antibody or an antigen binding fragment thereof can be the antibody against coatomer protein complex subunit beta 2 (COPB2) or the antigen binding fragment thereof according to (1), comprising any of the heavy chain variable regions and/or the light chain variable regions (d) to (f).
(3) A nucleic acid encoding the antibody or the antigen binding fragment thereof according to any of (1) to (2).
   In this paragraph, (1) to (2) means (1), (1-1), (1-2), and (2).
(4) An expression vector comprising the nucleic acid according to (3).
(5) A transformant comprising the expression vector according to (4).
(6) A kit for detecting COPB2 protein in a sample, comprising the antibody or the antigen binding fragment thereof according to (1) to (2).
(7) The kit according to (6), wherein the detection is based on an immunohistochemical method (IHC), an immunofluorescent method (IF), flow cytometry, enzyme-linked immunosorbent assay (ELISA), or immunoblot.

### Advantageous Effect of Invention

The present invention can provide a novel antibody capable of detecting COPB2 protein, or the like.

### Brief Description of Drawings

[Figure 1] Figure 1 shows the principle of measurement of an antibody titer after immunization of a mouse with purified human COPB2(650-906) protein.
[Figure 2] Figure 2 shows an antibody titer after two immunizations with purified human COPB2 protein in mice. A high antibody titer of 800000 counts or more was found in all A/Jackson mice, and an antibody titer of 200000 counts or less was found in Balb/c mice.

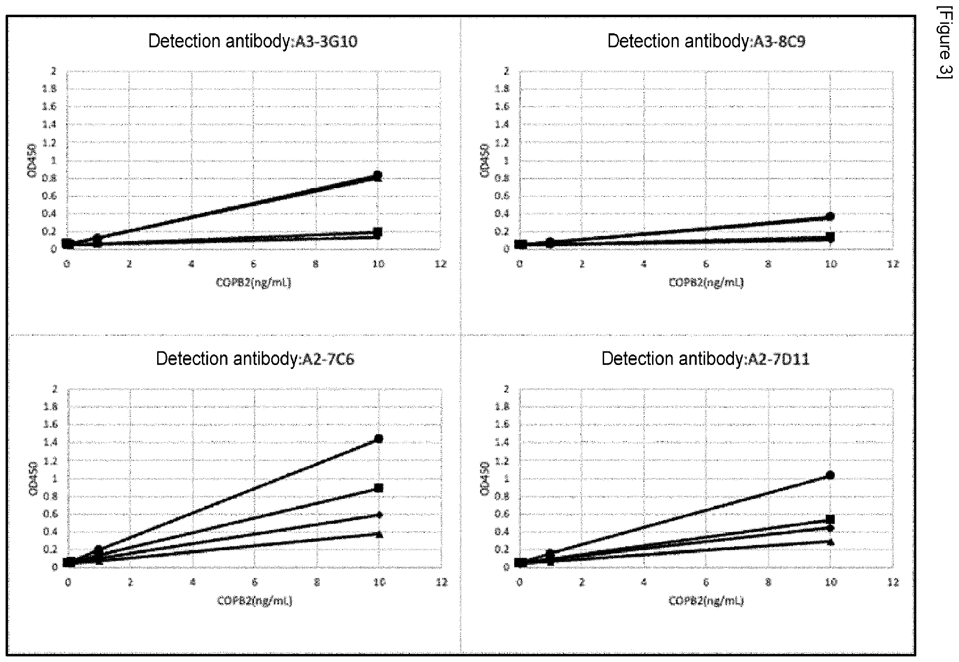

[Figure 3] Figure 3 shows a study on the combination of a solid-phase antibody and a labeled antibody in sandwich ELISA. Four panels are given on a detection antibody basis. Four line graphs in each panel depict absorbance (OD450) obtained on a solid-phase antibody basis. In the chart showing results obtained when the detection antibody was clone #A3-3G10, the values of OD450 (ordinate) show the results of clone #A2-7D11, clone #A2-7C6, clone #A3-8C9, and clone #A3-3G10 from top to bottom (in a descending order), and the values of clone #A2-7D11 and clone #A2-7C6 or the values of clone #A3-8C9 and clone #A3-3G10 are close to each other. In the chart showing results obtained when the detection antibody was clone #A3-8C9, the same results as in the case where the detection antibody was clone #A3-3G10 were obtained. In the chart showing results obtained when the detection antibody was clone #A2-7C6, the values of OD450 (ordinate) show the results of clone #A2-7D11, clone #A3-8C9, clone #A3-3G10, and clone #A2-7C6 from top to bottom (in a descending order ). In the chart showing results obtained when the detection antibody was clone #A2-7D11, the values of OD450 (ordinate) show the results about clone #A2-7C6, clone #A2-7D11, clone #A3-8C9, and clone #A3-3G10 from top to bottom (in a descending order). Regardless of which clone was used as the detection antibody, the values of OD450 (ordinate) were always in a relative relationship of clone #A2-7D11, clone #A3-8C9, and clone #A3-3G10 from top to bottom (in a descending order) and the order of clone #A2-7C6 differed relatively in the obtained results. With reference to the results obtained when the detection antibody was clone #A2-7C6, it was confirmed that the strongest signal was detected when clone #A2-7D11 was used as a solid-phase antibody and clone #A2-7C6 as a labeled antibody.
[Figure 4] Figure 4 shows a study on whether or not COPB2 protein in human cells and mouse cells can be detected by Western blot. Lane 1; 10 µg of a HEK293 cell extract, lane 2; 5 µg of a mouse liver extract, and lane 3; 10 µg of a mouse liver extract.
[Figure 5] Figure 5 shows results of immunostaining using BEAS-2B airway epithelial cells. The scale bar represents 50 µm. An antibody was used alone (Figure 5A), and both of an antibody and nuclear staining were used (Figure 5B). For all the antibodies, there was a location where stained regions accumulated near the nucleus, which morphologically seemed to be near the Golgi apparatus. A2-7C6 most clearly stained only the Golgi apparatus.
[Figure 6] Figure 6 shows results of immunostaining using a human lung cancer cell line NCI-H1975. The scale bar represents 50 µm. An antibody was used alone (Figure 6A), and both of an antibody and nuclear staining were used (Figure 6B). Slightly unlike the localization of BEAS-2B, spotted scattering was confirmed.
[Figure 7] Figure 7 shows the amino acid sequences of heavy chain CDR1 to CDR3 and light chain CDR1 to CDR3 (Figure 7A) and the amino acid sequences of a heavy chain variable region and a light chain variable region (Figure 7B) of clone #A2-7C6.
[Figure 8] Figure 8 shows the amino acid sequences of heavy chain CDR1 to CDR3 and light chain CDR1 to CDR3 (Figure 8A) and the amino acid sequences of a heavy chain variable region and a light chain variable region (Figure 8B) of clone #A2-7D11.
[Figure 9] Figure 9 shows the amino acid sequences of heavy chain CDR1 to CDR3 and light chain CDR1 to CDR3 (Figure 9A) and the amino acid sequences of a heavy chain variable region and a light chain variable region (Figure 9B) of clone #A3-8C9.

### Description of Embodiments

Hereinafter, embodiments of the present invention will be described. However, the present invention is not limited by the following illustration.

One aspect of the present embodiment is an antibody against coatomer protein complex subunit beta 2 (COPB2) or an antigen binding fragment thereof, comprising any of the following CDR sets (a) to (c):
(a)
   heavy chain CDR1 consisting of the amino acid sequence represented by SEQ ID NO: 1,
   heavy chain CDR2 consisting of the amino acid sequence represented by SEQ ID NO: 2,
   heavy chain CDR3 consisting of the amino acid sequence represented by SEQ ID NO: 3,
   light chain CDR1 consisting of the amino acid sequence represented by SEQ ID NO: 4,
   light chain CDR2 consisting of the amino acid sequence represented by SEQ ID NO: 5, and
   light chain CDR3 consisting of the amino acid sequence represented by SEQ ID NO: 6,
(b)
   heavy chain CDR1 consisting of the amino acid sequence represented by SEQ ID NO: 7,
   heavy chain CDR2 consisting of the amino acid sequence represented by SEQ ID NO: 8,
   heavy chain CDR3 consisting of the amino acid sequence represented by SEQ ID NO: 9,
   light chain CDR1 consisting of the amino acid sequence represented by SEQ ID NO: 10,
   light chain CDR2 consisting of the amino acid sequence represented by SEQ ID NO: 11, and
   light chain CDR3 consisting of the amino acid sequence represented by SEQ ID NO: 12,
(c)
   heavy chain CDR1 consisting of the amino acid sequence represented by SEQ ID NO: 13,
   heavy chain CDR2 consisting of the amino acid sequence represented by SEQ ID NO: 14,
   heavy chain CDR3 consisting of the amino acid sequence represented by SEQ ID NO: 15,
   light chain CDR1 consisting of the amino acid sequence represented by SEQ ID NO: 16,
   light chain CDR2 consisting of the amino acid sequence represented by SEQ ID NO: 17, and
   light chain CDR3 consisting of the amino acid sequence represented by SEQ ID NO: 18.

The antibody against coatomer protein complex subunit beta 2 (COPB2) or the antigen binding fragment thereof, having any of the CDR sets (a) to (c), specifically binds to COPB2 and is thereby capable of detecting the COPB2 protein.

In the present specification, the antibody against coatomer protein complex subunit beta 2 (COPB2) is also referred to as an anti-COPB2 antibody. Description of the anti-COPB2 antibody is also applied to the antigen binding fragment of the anti-COPB2 antibody. Thus, in the present specification, if no mention is made about the antigen binding fragment of the anti-COPB2 antibody, description of the anti-COPB2 antibody should be understood directly as description of the antigen binding fragment of the anti-COPB2 antibody.

The anti-COPB2 antibody is preferably an antibody comprising any of the CDR sets (a) to (c) and may be an antibody comprising any of the CDR sets (a1) to (c1) or may be an antibody comprising any of the CDR sets (a2) to (c2). Specifically, the description above should be understood as stating that the antigen binding fragment of the anti-COPB2 antibody is preferably an antigen binding fragment comprising any of the CDR sets (a) to (c) and may be an antigen binding fragment comprising any of the CDR sets (a1) to (c1) or may be an antigen binding fragment comprising any of the CDR sets (a2) to (c2).

COPB2 is a protein that is widely preserved from yeasts to humans. In one aspect, the anti-COPB2 antibody may be an antibody that specifically binds to human COPB2, and the antigen binding fragment of the anti-COPB2 antibody may also be an antigen binding fragment that specifically binds to human COPB2.

The term "antibody" as used herein means a molecule having a structure where pairs of two heavy chains (H chains) and two light chains (L chains) stabilized through a disulfide bond are associated with each other.

Two heavy chains are each composed of a heavy chain variable region VH, a heavy chain constant region CH1, CH2, and CH3, and a hinge region positioned between CH1 and CH2, and two light chains are each composed of a light chain variable region VL and a light chain constant region CL. In the antibody, a variable region fragment (Fv) composed of VH and VL is a region that is directly involved in binding to the antigen. An antigen binding region composed of VL, CL, VH, and CH1 is called Fab region, and a region composed of a hinge region, CH2, and CH3 is called Fc region.

In the variable regions, regions that come into direct contact with the antigen are largely variable and are called complementarity-determining regions (CDRs). Relatively less variable moieties other than CDRs are called framework regions (FRs). The light chain and heavy chain variable regions each have three CDRs (heavy chain CDR1 to CDR3 and light chain CDR1 to CDR3).

The antibody can be, for example, but not particularly limited to, a monoclonal antibody or a polyclonal antibody and is preferably a monoclonal antibody.

The term "monoclonal antibody" as used herein means an antibody that is obtained from a population of substantially homogeneous antibodies. The monoclonal antibody is produced by, for example, but not particularly limited to, a hybridoma method, a recombinant DNA method, a phage display method, or a method exploiting a recombinant animal containing the whole or a portion of human immunoglobulin loci, or a combined method thereof.

The term "polyclonal antibody" as used herein means a mixture of different antibodies that recognize a plurality of epitopes on one antigen. The polyclonal antibody is produced, for example, but not particularly limited to, by administering an immunogen comprising the antigen of interest to a mammal (e.g., a rat, a mouse, a rabbit, a bovine, or a monkey) or a bird (e.g., a chicken).

The anti-COPB2 antibody may be labeled and may be an antibody-drug conjugate (ADC) which is a conjugate with another drug.

The term "antigen binding fragment" as used herein means a molecule that comprises a portion of the antibody and is capable of binding to the same antigen as that for the antibody. Examples of the antigen binding fragment include, but are not particularly limited to, Fv, Fab, Fab', Fab'-SH, F(ab')2, diabody, linear antibodies, and single-chain antibody molecules (e.g., scFv).

The anti-COPB2 antibody can have a structure known for antibodies and can be any isotype of IgG, IgM, IgA, IgD, and IgE. IgG is an immunoglobulin having γ heavy chains and is produced as a portion of secondary immune response to the antigen. IgM is an immunoglobulin having µ heavy chains and exists as a pentamer in mammals. IgA is an immunoglobulin having α heavy chains. IgD is an immunoglobulin having ε heavy chains. IgE is an immunoglobulin having δ heavy chains.

The anti-COPB2 antibody can be a chimeric antibody, a humanized antibody, or the like. The antigen binding fragment of the anti-COPB2 antibody can be an antigen binding fragment of a chimeric antibody, an antigen binding fragment of a humanized antibody, or the like. The chimeric antibody is an antibody comprising linked fragments of antibodies derived from different species. The humanized antibody is an antibody comprising nonhuman CDR amino acid sequences grafted in a human antibody. Examples of the humanized antibody include, but are not particularly limited to, antibodies that have heavy chain CDR1 to CDR3 and light chain CDR1 to CDR3 of an antibody prepared by the immunization of a mouse or a rat and have other regions all derived from a human antibody. The term "humanized antibody" may also mean a human chimeric antibody.

One aspect of the present embodiment is an antibody against coatomer protein complex subunit beta 2 (COPB2) or an antigen binding fragment thereof, comprising any of the following CDR sets (a1) to (c1):
(a1)
   heavy chain CDR1 consisting of the amino acid sequence represented by SEQ ID NO: 1 or an amino acid sequence differing by having addition, substitution, or deletion of one to several amino acids from the amino acid sequence represented by SEQ ID NO: 1,
   heavy chain CDR2 consisting of the amino acid sequence represented by SEQ ID NO: 2 or an amino acid sequence differing by having addition, substitution, or deletion of one to several amino acids from the amino acid sequence represented by SEQ ID NO: 2,
   heavy chain CDR3 consisting of the amino acid sequence represented by SEQ ID NO: 3 or an amino acid sequence differing by having addition, substitution, or deletion of one to several amino acids from the amino acid sequence represented by SEQ ID NO: 3,
   light chain CDR1 consisting of the amino acid sequence represented by SEQ ID NO: 4 or an amino acid sequence differing by having addition, substitution, or deletion of one to several amino acids from the amino acid sequence represented by SEQ ID NO: 4,
   light chain CDR2 consisting of the amino acid sequence represented by SEQ ID NO: 5 or an amino acid sequence differing by having addition, substitution, or deletion of one to several amino acids from the amino acid sequence represented by SEQ ID NO: 5, and
   light chain CDR3 consisting of the amino acid sequence represented by SEQ ID NO: 6 or an amino acid sequence differing by having addition, substitution, or deletion of one to several amino acids from the amino acid sequence represented by SEQ ID NO: 6,
(b1)
   heavy chain CDR1 consisting of the amino acid sequence represented by SEQ ID NO: 7 or an amino acid sequence differing by having addition, substitution, or deletion of one to several amino acids from the amino acid sequence represented by SEQ ID NO: 7,
   heavy chain CDR2 consisting of the amino acid sequence represented by SEQ ID NO: 8 or an amino acid sequence differing by having addition, substitution, or deletion of one to several amino acids from the amino acid sequence represented by SEQ ID NO: 8,
   heavy chain CDR3 consisting of the amino acid sequence represented by SEQ ID NO: 9 or an amino acid sequence differing by having addition, substitution, or deletion of one to several amino acids from the amino acid sequence represented by SEQ ID NO: 9,
   light chain CDR1 consisting of the amino acid sequence represented by SEQ ID NO: 10 or an amino acid sequence differing by having addition, substitution, or deletion of one to several amino acids from the amino acid sequence represented by SEQ ID NO: 10,
   light chain CDR2 consisting of the amino acid sequence represented by SEQ ID NO: 11 or an amino acid sequence differing by having addition, substitution, or deletion of one to several amino acids from the amino acid sequence represented by SEQ ID NO: 11, and
   light chain CDR3 consisting of the amino acid sequence represented by SEQ ID NO: 12 or an amino acid sequence differing by having addition, substitution, or deletion of one to several amino acids from the amino acid sequence represented by SEQ ID NO: 12,
(c1)
   heavy chain CDR1 consisting of the amino acid sequence represented by SEQ ID NO: 13 or an amino acid sequence differing by having addition, substitution, or deletion of one to several amino acids from the amino acid sequence represented by SEQ ID NO: 13,
   heavy chain CDR2 consisting of the amino acid sequence represented by SEQ ID NO: 14 or an amino acid sequence differing by having addition, substitution, or deletion of one to several amino acids from the amino acid sequence represented by SEQ ID NO: 14,
   heavy chain CDR3 consisting of the amino acid sequence represented by SEQ ID NO: 15 or an amino acid sequence differing by having addition, substitution, or deletion of one to several amino acids from the amino acid sequence represented by SEQ ID NO: 15,
   light chain CDR1 consisting of the amino acid sequence represented by SEQ ID NO: 16 or an amino acid sequence differing by having addition, substitution, or deletion of one to several amino acids from the amino acid sequence represented by SEQ ID NO: 16,
   light chain CDR2 consisting of the amino acid sequence represented by SEQ ID NO: 17 or an amino acid sequence differing by having addition, substitution, or deletion of one to several amino acids from the amino acid sequence represented by SEQ ID NO: 17, and
   light chain CDR3 consisting of the amino acid sequence represented by SEQ ID NO: 18 or an amino acid sequence differing by having addition, substitution, or deletion of one to several amino acids from the amino acid sequence represented by SEQ ID NO: 18.

In the CDR sets (a1) to (c1), the heavy chain CDR1 to CDR3 and the light chain CDR1 to CDR3 may be of any of the CDR sets (a) to (c). In the CDR sets (a1) to (c1), the amino acid sequence of any CDR in (a1) to (c1) may be an amino acid sequence differing by having addition, substitution, or deletion of one to several amino acids from any of the amino acid sequences represented by SEQ ID NOs: 1 to 6, and the number of CDRs having the amino acid sequence having addition, substitution, or deletion of one to several amino acids can be 1 to 6.

In one aspect of the present embodiment, when the antibody against coatomer protein complex subunit beta 2 (COPB2) or the antigen binding fragment thereof comprises any of the CDR sets (a1) to (c1), any of the CDR sets is a set that has or retains the ability to bind to coatomer protein complex subunit beta 2 (COPB2).

When the phrase "having addition, substitution, or deletion of one to several amino acids" is used herein, the number of amino acids to be added, substituted, or deleted may be, for example, 1, 2, 3, 4, 5, 6, 7, 8, 9, or 10.

When the phrase "having addition, substitution, or deletion of one to several amino acids" is used concerning the amino acid sequence of CDR, the number of amino acids to be added, substituted, or deleted is not particularly limited as long as the anti-COPB2 antibody has binding activity against COPB2. The number can be set to, for example, 1, 2, 3, or 4. The amino acid sequence of CDR may have "addition, substitution, or deletion of one amino acid", may have "addition, substitution, or deletion of one or two amino acids", may have "addition, or deletion of one to three amino acids", and may have "addition, substitution, or deletion of one to four amino acids". The amino acid sequence of CDR preferably has "addition, substitution, or deletion of one or two amino acids" and preferably has "addition, substitution, or deletion of one amino acid".

The position of addition, substitution, or deletion of an amino acid may be any position in the amino acid sequence of CDR as long as binding activity against COPB2 is retained. The heavy chain and light chain variable regions of an antibody each comprise FR1, CDR1, FR2, CDR2, FR3, CDR3, and FR4 from the N terminus to the C terminus. Particularly, the heavy chain CDR3 is an important moiety that determines the antigen specificity of the antibody. The anti-COPB2 antibody, when having a mutation in CDR, may have addition, substitution, or deletion of one to several amino acids in CDR1 and/or CDR2 except for CDR3 and may have addition, substitution, or deletion of one amino acid therein, as long as the antibody retains antigen specificity.

One aspect of the present embodiment is an antibody against coatomer protein complex subunit beta 2 (COPB2) or an antigen binding fragment thereof, comprising any of the following CDR sets (a2) to (c2):
(a2)
   heavy chain CDR1 consisting of an amino acid sequence having 80% or higher identity to the amino acid sequence represented by SEQ ID NO: 1,
   heavy chain CDR2 consisting of an amino acid sequence having 80% or higher identity to the amino acid sequence represented by SEQ ID NO: 2,
   heavy chain CDR3 consisting of an amino acid sequence having 80% or higher identity to the amino acid sequence represented by SEQ ID NO: 3,
   light chain CDR1 consisting of an amino acid sequence having 80% or higher identity to the amino acid sequence represented by SEQ ID NO: 4,
   light chain CDR2 consisting of an amino acid sequence having 80% or higher identity to the amino acid sequence represented by SEQ ID NO: 5, and
   light chain CDR3 consisting of an amino acid sequence having 80% or higher identity to the amino acid sequence represented by SEQ ID NO: 6,
(b2)
   heavy chain CDR1 consisting of an amino acid sequence having 80% or higher identity to the amino acid sequence represented by SEQ ID NO: 7,
   heavy chain CDR2 consisting of an amino acid sequence having 80% or higher identity to the amino acid sequence represented by SEQ ID NO: 8,
   heavy chain CDR3 consisting of an amino acid sequence having 80% or higher identity to the amino acid sequence represented by SEQ ID NO: 9,
   light chain CDR1 consisting of an amino acid sequence having 80% or higher identity to the amino acid sequence represented by SEQ ID NO: 10,
   light chain CDR2 consisting of an amino acid sequence having 80% or higher identity to the amino acid sequence represented by SEQ ID NO: 11, and
   light chain CDR3 consisting of an amino acid sequence having 80% or higher identity to the amino acid sequence represented by SEQ ID NO: 12,
(c2)
   heavy chain CDR1 consisting of an amino acid sequence having 80% or higher identity to the amino acid sequence represented by SEQ ID NO: 13,
   heavy chain CDR2 consisting of an amino acid sequence having 80% or higher identity to the amino acid sequence represented by SEQ ID NO: 14,
   heavy chain CDR3 consisting of an amino acid sequence having 80% or higher identity to the amino acid sequence represented by SEQ ID NO: 15,
   light chain CDR1 consisting of an amino acid sequence having 80% or higher identity to the amino acid sequence represented by SEQ ID NO: 16,
   light chain CDR2 consisting of an amino acid sequence having 80% or higher identity to the amino acid sequence represented by SEQ ID NO: 17, and
   light chain CDR3 consisting of an amino acid sequence having 80% or higher identity to the amino acid sequence represented by SEQ ID NO: 18.

In the CDR sets (a2) to (c2), the heavy chain CDR1 to CDR3 and the light chain CDR1 to CDR3 may be of any of the CDR sets (a) to (c). In the CDR sets (a2) to (c2), which are not any of the CDR sets (a) to (c), the amino acid sequence of any CDR in (a) to (c) may be an amino acid sequence having 80% or higher identity to any of the amino acid sequences represented by SEQ ID NOs: 1 to 6, and the number of CDRs having the amino acid sequence having 80% or higher identity can be 1 to 6.

In one aspect of the present embodiment, when the antibody against coatomer protein complex subunit beta 2 (COPB2) or the antigen binding fragment thereof comprises any of the CDR sets (a2) to (c2), any of the CDR sets is a set that has or retains the ability to bind to coatomer protein complex subunit beta 2 (COPB2).

The phrase "having 80% or higher identity" as used herein means that when an amino acid sequence before mutation is aligned with an amino acid sequence mutated from the amino acid sequence so as to maximize matches, the number of common amino acid residues is 80% or more of the number of amino acids of the amino acid sequence before mutation.

The identity can be, for example, 85% or higher, 90% or higher, 95% or higher, 96% or higher, 97% or higher, 98% or higher, or 99% or higher. The identity may be 100%.

A set of heavy chain CDR1 to CDR3 and light chain CDR1 to CDR3 each consisting of only an amino acid sequence having 100% identity is any of the CDR sets (a) to (c).

One aspect of the present embodiment is an antibody against coatomer protein complex subunit beta 2 (COPB2) or an antigen binding fragment thereof, comprising any of heavy chain variable regions and/or light chain variable regions (d) to (f) given below.

The heavy chain variable regions and/or the light chain variable regions (d) to (f) given below preferably have any of the CDR sets described above and more preferably have any of the CDR sets (a) to (c).

The case of (d) will be described as an example (the same holds true for (e) and (f)). The heavy chain variable region and/or the light chain variable region (d) is any heavy chain variable region described in (d), is any light chain variable region described in (d), or is any heavy chain variable region described in (d) and any light chain variable region described in (d).

The anti-COPB2 antibody preferably comprises any of the following heavy chain variable regions and light chain variable regions (d) to (f), and, for example, in the case of (d), more preferably comprises any heavy chain variable region (d) and any light chain variable region (d):
(d)
   a heavy chain variable region comprising the amino acid sequence represented by SEQ ID NO: 19;
   a heavy chain variable region comprising an amino acid sequence differing by having addition, substitution, or deletion of one to several amino acids from the amino acid sequence represented by SEQ ID NO: 19; or
   a heavy chain variable region comprising an amino acid sequence having 80% or higher identity to the amino acid sequence represented by SEQ ID NO: 19,
   a light chain variable region comprising the amino acid sequence represented by SEQ ID NO: 20;
   a light chain variable region comprising an amino acid sequence differing by having addition, substitution, or deletion of one to several amino acids from the amino acid sequence represented by SEQ ID NO: 20; or
   a light chain variable region comprising an amino acid sequence having 80% or higher identity to the amino acid sequence represented by SEQ ID NO: 20,
(e)
   a heavy chain variable region comprising the amino acid sequence represented by SEQ ID NO: 21;
   a heavy chain variable region comprising an amino acid sequence differing by having addition, substitution, or deletion of one to several amino acids from the amino acid sequence represented by SEQ ID NO: 21; or
   a heavy chain variable region comprising an amino acid sequence having 80% or higher identity to the amino acid sequence represented by SEQ ID NO: 21,
   a light chain variable region comprising the amino acid sequence represented by SEQ ID NO: 22;
   a light chain variable region comprising an amino acid sequence differing by having addition, substitution, or deletion of one to several amino acids from the amino acid sequence represented by SEQ ID NO: 22; or
   a light chain variable region comprising an amino acid sequence having 80% or higher identity to the amino acid sequence represented by SEQ ID NO: 22,
(f)
   a heavy chain variable region comprising the amino acid sequence represented by SEQ ID NO: 23;
   a heavy chain variable region comprising an amino acid sequence differing by having addition, substitution, or deletion of one to several amino acids from the amino acid sequence represented by SEQ ID NO: 23; or
   a heavy chain variable region comprising an amino acid sequence having 80% or higher identity to the amino acid sequence represented by SEQ ID NO: 23,
   a light chain variable region comprising the amino acid sequence represented by SEQ ID NO: 24;
   a light chain variable region comprising an amino acid sequence differing by having addition, substitution, or deletion of one to several amino acids from the amino acid sequence represented by SEQ ID NO: 24; or
   a light chain variable region comprising an amino acid sequence having 80% or higher identity to the amino acid sequence represented by SEQ ID NO: 24.

The heavy chain and light chain variable regions have framework regions (FRs) in addition to CDRs. The amino acid sequence of each FR may be the amino acid sequence itself derived from the FR in each variable region of each immunoglobulin, may be a sequence having a mutation in the amino acid sequence, or may be partially engineered, for example, by introducing a restriction enzyme recognition site into a portion of the amino acid sequence derived from the FR. In the amino acid sequence of the heavy chain and/or light chain variable region, an amino acid mutation may be present in a region other than CDR, i.e., FR, from the viewpoint of less influencing reactivity with the antigen.

The anti-COPB2 antibody may comprise the following CDR set:
heavy chain CDR1 consisting of the amino acid sequence represented by SEQ ID NO: 1,
heavy chain CDR2 consisting of the amino acid sequence represented by SEQ ID NO: 2,
heavy chain CDR3 consisting of the amino acid sequence represented by SEQ ID NO: 3,
light chain CDR1 consisting of the amino acid sequence represented by SEQ ID NO: 4,
light chain CDR2 consisting of the amino acid sequence represented by SEQ ID NO: 5, and
light chain CDR3 consisting of the amino acid sequence represented by SEQ ID NO: 6, and
comprise any following heavy chain variable region and/or any following light chain variable region:
a heavy chain variable region comprising the amino acid sequence represented by SEQ ID NO: 19;
a heavy chain variable region comprising an amino acid sequence differing by having addition, substitution, or deletion of one to several amino acids from the amino acid sequence represented by SEQ ID NO: 19; or
a heavy chain variable region comprising an amino acid sequence having 80% or higher identity to the amino acid sequence represented by SEQ ID NO: 19, and/or
a light chain variable region comprising the amino acid sequence represented by SEQ ID NO: 20;
a light chain variable region comprising an amino acid sequence differing by having addition, substitution, or deletion of one to several amino acids from the amino acid sequence represented by SEQ ID NO: 20; or
a light chain variable region comprising an amino acid sequence having 80% or higher identity to the amino acid sequence represented by SEQ ID NO: 20.

The anti-COPB2 antibody may comprise the following CDR set:
heavy chain CDR1 consisting of the amino acid sequence represented by SEQ ID NO: 7,
heavy chain CDR2 consisting of the amino acid sequence represented by SEQ ID NO: 8,
heavy chain CDR3 consisting of the amino acid sequence represented by SEQ ID NO: 9,
light chain CDR1 consisting of the amino acid sequence represented by SEQ ID NO: 10,
light chain CDR2 consisting of the amino acid sequence represented by SEQ ID NO: 11, and
light chain CDR3 consisting of the amino acid sequence represented by SEQ ID NO: 12, and
comprise any following heavy chain variable region and/or any following light chain variable region:
a heavy chain variable region comprising the amino acid sequence represented by SEQ ID NO: 21;
a heavy chain variable region comprising an amino acid sequence differing by having addition, substitution, or deletion of one to several amino acids from the amino acid sequence represented by SEQ ID NO: 21; or
a heavy chain variable region comprising an amino acid sequence having 80% or higher identity to the amino acid sequence represented by SEQ ID NO: 21, and/or
a light chain variable region comprising the amino acid sequence represented by SEQ ID NO: 22;
a light chain variable region comprising an amino acid sequence differing by having addition, substitution, or deletion of one to several amino acids from the amino acid sequence represented by SEQ ID NO: 22; or
a light chain variable region comprising an amino acid sequence having 80% or higher identity to the amino acid sequence represented by SEQ ID NO: 22.

The anti-COPB2 antibody may comprise the following CDR set:
heavy chain CDR1 consisting of the amino acid sequence represented by SEQ ID NO: 13,
heavy chain CDR2 consisting of the amino acid sequence represented by SEQ ID NO: 14,
heavy chain CDR3 consisting of the amino acid sequence represented by SEQ ID NO: 15,
light chain CDR1 consisting of the amino acid sequence represented by SEQ ID NO: 16,
light chain CDR2 consisting of the amino acid sequence represented by SEQ ID NO: 17, and
light chain CDR3 consisting of the amino acid sequence represented by SEQ ID NO: 18, and
comprise any following heavy chain variable region and/or any following light chain variable region:
a heavy chain variable region comprising the amino acid sequence represented by SEQ ID NO: 23;
a heavy chain variable region comprising an amino acid sequence differing by having addition, substitution, or deletion of one to several amino acids from the amino acid sequence represented by SEQ ID NO: 23; or
a heavy chain variable region comprising an amino acid sequence having 80% or higher identity to the amino acid sequence represented by SEQ ID NO: 23, and/or
a light chain variable region comprising the amino acid sequence represented by SEQ ID NO: 24;
a light chain variable region comprising an amino acid sequence differing by having addition, substitution, or deletion of one to several amino acids from the amino acid sequence represented by SEQ ID NO: 24; or
a light chain variable region comprising an amino acid sequence having 80% or higher identity to the amino acid sequence represented by SEQ ID NO: 24.

In the anti-COPB2 antibody, regions other than CDRs when the CDRs are defined or regions other than the variable regions when a heavy chain and/or light chain variable region are defined may be selected from regions of usual antibodies as long as the anti-COPB2 antibody has the ability to bind to COPB2.

The heavy chain and light chain variable regions in the anti-COPB2 antibody may have amino acid sequences differing from the amino acid sequences represented by SEQ ID NOs: 19 to 24 by lacking amino acids constituting a signal sequence.

Specifically, the anti-COPB2 antibody may comprise
a heavy chain variable region comprising an amino acid sequence differing from the amino acid sequence represented by any of SEQ ID NOs: 19, 21, and 23 by lacking amino acids constituting a signal sequence, and/or
a light chain variable region comprising an amino acid sequence differing from the amino acid sequence represented by any of SEQ ID NOs: 20, 22, and 24 by lacking amino acids constituting a signal sequence.

The heavy chain or light chain variable region comprising an amino acid sequence having addition, substitution, or deletion of one to several amino acids can also be a heavy chain or light chain variable region comprising an amino acid sequence differing by having addition, substitution, or deletion of one to several amino acids from an amino acid sequence differing from the amino acid sequence represented by any of SEQ ID NOs: 19 to 24 by lacking amino acids constituting a signal sequence.

The heavy chain or light chain variable region having an amino acid sequence having 80% or higher identity can also be a heavy chain or light chain variable region comprising an amino acid sequence having 80% or higher identity to an amino acid sequence differing from the amino acid sequence represented by any of SEQ ID NOs: 19 to 24 by lacking amino acids constituting a signal sequence.

The signal sequence corresponds to approximately 20 amino acids from an N-terminal amino acid in the heavy chain and light chain variable regions.

The amino acid sequence corresponding to the signal sequence is as follows:
positions 1 to 28 of SEQ ID NO: 19,
positions 1 to 36 of SEQ ID NO: 20,
positions 1 to 28 of SEQ ID NO: 21,
positions 1 to 22 of SEQ ID NO: 22,
positions 1 to 19 of SEQ ID NO: 23, and
positions 1 to 19 of SEQ ID NO: 24.

Thus, the antibody against coatomer protein complex subunit beta 2 (COPB2) or the antigen binding fragment thereof, comprising any of the heavy chain variable regions and/or the light chain variable regions (d) to (f) may be an antibody against coatomer protein complex subunit beta 2 (COPB2) or an antigen binding fragment thereof, comprising any of the following heavy chain variable regions and/or light chain variable regions (d1) to (f1) as an antibody or an antigen binding fragment thereof comprising no signal sequence:
(d1)
   a heavy chain variable region comprising the amino acid sequence represented by positions 29 to 145 of SEQ ID NO: 19;
   a heavy chain variable region comprising an amino acid sequence differing by having addition, substitution, or deletion of one to several amino acids from the amino acid sequence represented by positions 29 to 145 of SEQ ID NO: 19; or
   a heavy chain variable region comprising an amino acid sequence having 80% or higher identity to the amino acid sequence represented by positions 29 to 145 of SEQ ID NO: 19, and/or
   a light chain variable region comprising the amino acid sequence represented by positions 37 to 143 of SEQ ID NO: 20;
   a light chain variable region comprising an amino acid sequence differing by having addition, substitution, or deletion of one to several amino acids from the amino acid sequence represented by positions 37 to 143 of SEQ ID NO: 20; or
   a light chain variable region comprising an amino acid sequence having 80% or higher identity to the amino acid sequence represented by positions 37 to 143 of SEQ ID NO: 20,
(e1)
   a heavy chain variable region comprising the amino acid sequence represented by positions 29 to 145 of SEQ ID NO: 21;
   a heavy chain variable region comprising an amino acid sequence differing by having addition, substitution, or deletion of one to several amino acids from the amino acid sequence represented by positions 29 to 145 of SEQ ID NO: 21; or
   a heavy chain variable region comprising an amino acid sequence having 80% or higher identity to the amino acid sequence represented by positions 29 to 145 of SEQ ID NO: 21, and/or
   a light chain variable region comprising the amino acid sequence represented by positions 23 to 129 of SEQ ID NO: 22;
   a light chain variable region comprising an amino acid sequence differing by having addition, substitution, or deletion of one to several amino acids from the amino acid sequence represented by positions 23 to 129 of SEQ ID NO: 22; or
   a light chain variable region comprising an amino acid sequence having 80% or higher identity to the amino acid sequence represented by positions 23 to 129 of SEQ ID NO: 22,
(f1)
   a heavy chain variable region comprising the amino acid sequence represented by positions 20 to 134 of SEQ ID NO: 23;
   a heavy chain variable region comprising an amino acid sequence differing by having addition, substitution, or deletion of one to several amino acids from the amino acid sequence represented by positions 20 to 134 of SEQ ID NO: 23; or
   a heavy chain variable region comprising an amino acid sequence having 80% or higher identity to the amino acid sequence represented by positions 20 to 134 of SEQ ID NO: 23, and/or
   a light chain variable region comprising the amino acid sequence represented by positions 20 to 131 of SEQ ID NO: 24;
   a light chain variable region comprising an amino acid sequence differing by having addition, substitution, or deletion of one to several amino acids from the amino acid sequence represented by positions 20 to 131 of SEQ ID NO: 24; or
   a light chain variable region comprising an amino acid sequence having 80% or higher identity to the amino acid sequence represented by positions 20 to 131 of SEQ ID NO: 24.

The antibody against coatomer protein complex subunit beta 2 (COPB2) or the antigen binding fragment thereof, comprising any of the heavy chain variable regions and/or the light chain variable regions (d1) to (f1) is preferably the antibody or the antigen binding fragment thereof according to any of (1) to (1-2).

Specifically, the antibody against coatomer protein complex subunit beta 2 (COPB2) or the antigen binding fragment thereof, comprising any of heavy chain variable regions and/or light chain variable regions (d1) to (f1), when being an antibody against coatomer protein complex subunit beta 2 (COPB2) or an antigen binding fragment thereof, comprising the heavy chain variable region and/or the light chain variable region (d1), preferably comprises any of the CDR set (a), (a1), or (a2) and more preferably comprises the CDR set (a); when being an antibody against coatomer protein complex subunit beta 2 (COPB2) or an antigen binding fragment thereof, comprising the heavy chain variable region and/or the light chain variable region (e1), preferably comprises any of the CDR set (b), (b1), or (b2) and more preferably comprises the CDR set (b); and when being an antibody against coatomer protein complex subunit beta 2 (COPB2) or an antigen binding fragment thereof, comprising the heavy chain variable region and/or the light chain variable region (f1), preferably comprises any of the CDR set (c), (c1), or (c2) and more preferably comprises the CDR set (c).

Such an antibody or an antigen binding fragment thereof can be the antibody against coatomer protein complex subunit beta 2 (COPB2) or the antigen binding fragment thereof according to (1), comprising any of the heavy chain variable regions and/or the light chain variable regions (d1) to (f1).

The description of the heavy chain variable regions and/or the light chain variable regions (d) to (f) should be understood as description of the heavy chain variable regions and/or the light chain variable regions (d1) to (f1). In the following description of a nucleic acid, an expression vector, a transformant, a production method, a kit, and the like in the present specification, the description of the heavy chain variable regions and/or the light chain variable regions (d) to (f) should also be understood as description of the heavy chain variable regions and/or the light chain variable regions (d1) to (f1).

### (Nucleic acid)

One aspect of the present embodiment is a nucleic acid encoding the antibody against COPB2 or the antigen binding fragment thereof.

The nucleic acid is not particularly limited and may be a natural nucleic acid or may be an artificial nucleic acid. Examples thereof include DNA, RNA, and chimeric nucleic acids of DNA and RNA. The nucleotide sequence of the nucleic acid encoding the anti-COPB2 antibody or the antigen binding fragment thereof is determined depending on the amino acid sequence, and the nucleic acid can be prepared by a method known to those skilled in the art or a method equivalent thereto.

The nucleic acid may be present in a single vector or separate vectors or may be present at one or more locations in a host cell. The nucleic acid encoding the anti-COPB2 antibody or the antigen binding fragment thereof can be one or more nucleic acid molecules encoding heavy and light chains of the antibody or an antigen binding fragment thereof.

### (Expression vector)

One aspect of the present embodiment is an expression vector comprising the nucleic acid.

The expression vector is not particularly limited and can be appropriately selected depending on the host cell used. Examples thereof include plasmids, retrovirus vectors, adenovirus vectors, adeno-associated virus (AAV) vectors, plant virus vectors (e.g., cauliflower mosaic virus vectors and tobacco mosaic virus vectors), cosmids, YAC, and EBV-derived episome. The nucleic acid may be inserted into such an expression vector by a method known in the art (e.g., a method using a restriction enzyme) or a method equivalent thereto to prepare an expression vector comprising the nucleic acid.

The expression vector can further comprise a promoter that regulates the expression of the nucleic acid gene of the antibody against COPB2 or the antigen binding fragment thereof, a replication origin, a selective marker gene, and the like, in addition to the nucleic acid encoding the antibody against COPB2 or the antigen binding fragment thereof. The promoter and the replication origin can be appropriately selected depending on the types of the host cell and the vector.

### (Transformant)

One aspect of the present embodiment is a transformant comprising the vector.

The transformant is not particularly limited and may be prepared by transfecting an appropriate host cell with the expression vector. Specifically, the transformant can be a cell (host cell) comprising the expression vector. Examples of the host cell include, but are not particularly limited to, eukaryotic cells such as mammalian cells (CHO cells, COS cells, myeloma cells, HeLa cells, Vero cells, etc.), insect cells, plant cells, and fungal cells (the genus *Saccharomyces,* the genus *Aspergillus*, etc.), and prokaryotic cells such as *E*. *coli* and *Bacillus subtilis.*

### (Method for producing antibody against COPB2 or antigen binding fragment thereof)

The method for producing the anti-COPB2 antibody or the antigen binding fragment thereof is not particularly limited. In the case of, for example, a monoclonal antibody, the method can involve isolating antibody-producing cells from a nonhuman mammal immunized with COPB2 or its fragment, fusing the cells with myeloma cells or the like to prepare hybridomas, and purifying antibodies produced from the hybridomas. A polyclonal antibody may be obtained from the serum of an animal immunized with COPB2 or its fragment.

In the case of preparing the anti-COPB2 antibody or the antigen binding fragment thereof by a gene recombination method, for example, appropriate host cells are transformed with the expression vector comprising the nucleic acid encoding the anti-COPB2 antibody or the antigen binding fragment thereof, and the obtained transformants are cultured under appropriate conditions to express the antibody or the antigen binding fragment. Then, isolation or purification may be performed by a method known in the art or a method equivalent thereto.

Examples of the method for isolating or purifying the antibody or the antigen binding fragment include, but are not particularly limited to, column purification using affinity columns with protein A or other chromatographic columns, filtration through filters, ultrafiltration, salting-out, and dialysis, which may be appropriately used in combination.

When the anti-COPB2 antibody has CDRs consisting of amino acid sequences derived from the amino acid sequences of heavy chain CDR1 to CDR3 and light chain CDR1 to CDR3 by the addition, substitution, or deletion of an amino acid or has CDRs consisting of amino acid sequences having 80% or higher identity to the amino acid sequences of heavy chain CDR1 to CDR3 and light chain CDR1 to CDR3, the anti-COPB2 antibody can be prepared by use of a method known in the art such as site-directed mutagenesis, random mutagenesis, chain shuffling, or CDR walking.

It is well known to those skilled in the art that antibodies or antigen binding fragments thereof having various mutations in CDRs are displayed on phage surface by a phage display method and screened using the antigen to obtain an antibody or an antigen binding fragment thereof comprising a CDR set with more matured affinity (e.g., Wu et al., PNAS, 95: 6037-6042 (1998); Schier, R. et al., J. Mol. Bio. 263: 551-567 (1996); Schier, R. et al., J. Mol. Biol. 255: 28-43 (1996); and Yang, W.P. et al., J. Mol. Biol., 254: 392-403 (1995)). Thus, the anti-COPB2 antibody may be prepared by this method.

A chimeric antibody and a humanized antibody can be prepared by methods known in the art or methods equivalent thereto.

The chimeric antibody and the humanized antibody may be prepared by cloning an antibody gene from mRNA of a hybridoma producing a nonhuman animal antibody, linking this antibody gene to a portion of a human antibody gene by a gene recombination technique, and using the resulting gene.

The gene of the anti-COPB2 antibody may be determined depending on the amino acid sequences of the CDRs or the amino acid sequences of the variable regions described in (a) to (f).

The preparation of the chimeric antibody is not particularly limited, and the chimeric antibody may be produced using, for example, a nucleic acid obtained by substituting any of the heavy chain variable regions (VH) and the light chain variable regions (LH) (d) to (f) with human antibody-derived sequences by gene recombination.

Alternatively, the chimeric antibody may be obtained by cloning heavy chain and light chain constant regions of human IgG1, linking these human antibody-derived heavy chain and light chain constant regions to, for example, any of the heavy chain variable regions (VH) and the light chain variable regions (LH (d) to (f), respectively, by an overlapping hanging method based on PCR, and inserting DNA obtained after amplification into an appropriate vector, which is then used in transformation.

In the preparation of the humanized antibody, human antibody variable domains are cloned and engineered so as to have the nucleotide sequences of the CDRs of the anti-COPB2 antibody by site-directed mutagenesis using a megaprimer method. If specific binding to the antigen can no longer be attained by humanizing amino acid sequences constituting framework regions, some amino acids of the framework regions may be converted from human type to rat type.

CDRs consisting of amino acid sequences differing by having deletion, substitution, or addition of one or more amino acids from the amino sequences of the original CDRs or CDRs consisting of amino acid sequences having 80% or higher identity to the original sequences may be prepared by use of a method known in the art such as site-directed mutagenesis, random mutagenesis, chain shuffling, or CDR walking.

The antigen binding fragment of the anti-COPB2 antibody may be expressed by the method mentioned above using the nucleic acid encoding the fragment, or the full-length antibody may be obtained and then fragmented by treatment with an enzyme such as papain or pepsin.

The amount of the COPB2 protein in a COVID-19 patient can be measured through the use of the anti-COPB2 antibody. For example, the abundance of the COPB2 protein in a biological sample collected from a COVID-19 patient can be determined by a method for detecting the abundance and compared with a biological sample of a patient not infected with COVID-19 to predict a worsening tendency of COVID-19.

### (Kit)

One aspect of the present embodiment is a kit for detecting COPB2 protein in a sample,
the kit comprising the antibody against COPB2 or the antigen binding fragment thereof.

The antibody against COPB2 or the antigen binding fragment thereof contained in the kit can employ the anti-COPB2 antibody or the antigen binding fragment thereof described in the present specification.

An instruction regarding use of the kit may be attached to the kit.

The kit may comprise, for example, a reagent necessary for detecting the COPB2 protein in the sample, in addition to the anti-COPB2 antibody or the antigen binding fragment thereof.

Examples of the reagent include, but are not particularly limited to, antigen retrieval agents, enzymatic antigen retrieval agents, blocking agents, washing solutions, fluorescent dyes, labeling enzymes, labeled secondary antibodies, staining agents, coloring agents, fixation solutions for cells, or the like, membrane permeation treatment agents, dehydrating agents, mountants, detecting agents, reaction terminating solutions, buffer solutions, cell lysis buffer solutions, loading buffer solutions, running buffer solutions and transfer buffer solutions. The kit may comprise at least one of these reagents.

The sample from which the COPB2 protein is to be detected using the anti-COPB2 antibody or the antigen binding fragment thereof is not particularly limited and can be, for example, a biological sample separated from an animal such as a human. A nonhuman mammal is not particularly limited and may be a rodent such as a mouse or a rat or may be a dog, a bovine, a horse, a monkey, a rabbit, a pig, or the like.

Examples of the biological sample include, but are not particularly limited to, tracheal swabs, oral swabs, nasal swabs, pharyngeal swabs, nasal lavages, oral lavages, nasal aspirates, saliva, sputum, blood, serum, urine, tissues, cells, and homogenates of tissues or cells. Among them, blood or serum is preferred.

Since COPB2 is also known to exist in exosome, the COPB2 protein in exosome may be a target for detection. The anti-COPB2 antibody or the antigen binding fragment thereof may be used for detecting exosome in a sample via the detection of the COPB2 protein. The exosome is secreted from cells, not only resides between cells but resides in body fluids (blood, spinal fluid, urine, etc.), and is circulated throughout the body. Examples of the biological sample for use in the detection of exosome or the detection of the COPB2 protein in exosome include, but are not particularly limited to, body fluids.

In the case of using blood as a biological sample, for example, but not particularly limited to, whole blood, plasma, or serum may be used directly as the sample, or a treated material obtained by the treatment of blood, for example, may be used for hemolysis treatment or for obtaining particular fractions.

The sample may be an appropriately diluted or concentrated biological sample. The sample may comprise, for example, but not particularly limited to, a pH adjuster, glycine, polysaccharides, sodium azide, thimerosal, EDTA, DTT, 2-mercaptoethanol, bovine serum albumin, glycerol, or ethylene glycol, for stable existence of the COPB2 protein.

The detection of the COPB2 protein can be carried out, for example, but not particularly limited to, by detecting the COPB2 protein in the sample by an immunological measurement method using the anti-COPB2 antibody or the antigen binding fragment thereof.

Immunoassay for detecting the protein of interest by immunological measurement is well known in the art.

Reagents necessary for each immunoassay are also well known. The COPB2 protein may be immunologically detected using the anti-COPB2 antibody or the antigen binding fragment thereof as an antibody for use in immunoassay, and using a usual immunoassay kit.

The immunoassay is a method for qualitatively and quantitatively analyzing a minor component through the use of specific antigen-antibody reaction. The antigen-antibody reaction is widely used due to its high sensitivity or reaction selectivity and includes various measurement methods depending on the measurement principle.

Examples of the immunoassay include, but are not particularly limited to, methods shown in the table below.

**[Table 1]**

| | Labeling agent | Measurement object | Application example |
|---|---|---|---|
| Labeling method | Enzyme | Enzymatic activity (color development/fluorescence/ luminescence, etc.) | Enzyme-linked immunosorbent assay (ELIZA), immunoblot |
| | Phosphor | Fluorescence intensity, delayed fluorescence, fluorescence polarization | Immunohistochemical method (IHC), flow cytometry, immunoblot, immunofluorescent method (IF) |
| | Luminant | Chemiluminescence, electrochemiluminescence | Immunoblot, chemiluminescent immunoassay, chemiluminescent enzyme immunoassay |
| | Chromogen | Absorbance | Enzyme-linked immunosorbent assay (ELIZA) |
| | Metal | Light/electrochemistry | Immunoelectron microscopy |
| | Spin (radical species) | ESR | Spin labeling method |
| | Magnetic body | Magnetism | Immunoprecipitation (IP) |
| | Electrochemically active body | Current/potential | Immunosensor |
| | Radioactive material | Radioactivity intensity | Immunoblot, immunoradiometric assay |
| Non-labeling method | None | Light scattering/turbidity, SPR/LSPR | Nephelometry, latex agglutination turbidimetry |
| | | Mass change (piezoelectric element/AFM) | Latex piezoelectric immunoassay |
| | | Electrochemical impedance, etc. | Electrochemical impedance method |

The immunoassay is not particularly limited and is preferably, for example, an immunohistochemical method (IHC), an immunofluorescent method (IF), flow cytometry, enzyme-linked immunosorbent assay (ELISA), or immunoblot.

A disease (cancer or infection, etc.) may be diagnosed by the detection of the COPB2 protein. The kit comprising the anti-COPB2 antibody may be used as a tool for performing such diagnosis.

For example, the anti-COPB2 antibody or the antigen binding fragment thereof may be used for predicting the development of a cancer or predicting the progression thereof, for predicting the possibility of cancer metastasis, or for diagnosing cancer prognosis or predicting the possibility of recurrence.

The disclosure of all patent literatures and non-patent literatures cited herein is incorporated herein by reference in its entirety.

### Examples

Some examples of the embodiments of the present application will be described below.

Hereinafter, the present invention will be specifically described with reference to Examples. However, the present invention is not limited by these Examples.

### 1. Preparation of antigen protein

cDNA was synthesized such that a 6 x His tag is connected downstream of cDNA encoding 255 amino acid residues corresponding to glutamine at position 650 to aspartic acid at position 906 in the amino acid sequence of human COPB2. This cDNA was cloned into an expression vector pcDNA5/TO (Thermo Fisher Scientific Inc.). Expi293 cells (Thermo Fisher Scientific Inc.) were transfected with this plasmid so that human COPB2 antigen protein was expressed. The transfection was performed using Expi293fectamine (Thermo Fisher Scientific Inc.) in accordance with the method described in the attached document. A culture solution and the cells were each recovered 6 days after transfection, and a cell extract was prepared from the cells using RIPA (Invitrogen Corp.). 10 µL of the culture solution and 10 µg of the cell extract were subjected to SDS-PAGE under reducing conditions, and the expression of COPB2(650-906) was confirmed by Western blot using an anti-His tag antibody (Proteintech Group, Inc.).

As a result, the expression of COPB2(650-906) was found in both the culture solution and the cells, and it was confirmed that more COPB2(650-906) was expressed and accumulated in the cells.

The purification of the COPB2(650-906) protein from the culture solution of the transfected Expi293 cells and the cells was carried out using TALON resin (Clontech Laboratories, Inc.). By two transfection experiments, 524 µg in total of purified COPB2(650-906) protein was obtained from the culture solution and the cells.

### 2. Preparation of monoclonal antibody

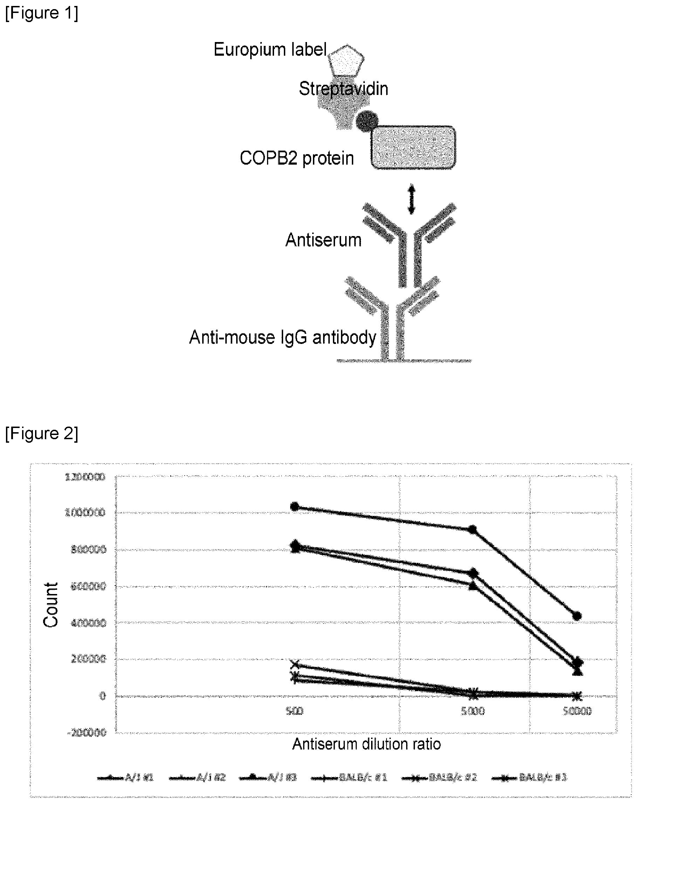

Three A/Jackson mice and three Balb/c mice were immunized with the purified human COPB2(650-906) protein. Specifically, 50 µg/shot of the purified human COPB2(650-906) protein was administered intraperitoneally and 50 µg/shot of the purified human COPB2(650-906) protein was administered subcutaneously to the neck, respectively, per mouse. This immunization was carried out twice every three weeks. After the two immunizations, blood was collected, and an antibody titer against the antigen protein was measured. The principle of the measurement method is shown in Figure 1.

As a result, a high antibody titer of 800000 counts or more was found in all the A/Jackson mice immunized with the human COPB2 protein. On the other hand, an antibody titer of 200000 counts or less was found in the Balb/c mice (Figure 2).

One A/Jackson mouse (A/J#3 mouse) that exhibited a high antibody titer after two immunizations with the purified human COPB2 protein was given the antigen protein as a final booster, and the spleen was excised 4 days later. Spleen cells were prepared and subsequently subjected to cell fusion with myeloma cells (P3U1). Hybridomas were inoculated to ten 96-well plates, and an antibody titer against the immunogen human COPB2 protein was measured for culture supernatants in a total of 928 wells. As a result, five positive samples were confirmed. The cells contained in the wells of these five positive samples were further subjected to the limiting dilution method twice to obtain monoclonal lines. As a result, hybridomas of two clones were established as cell lines producing antibodies that exhibited high reactivity with the COPB2 protein (clone #A3-3G10 and #A3-8C9).

Further, spleen cells were also prepared from the mouse (A/J#2 mouse) that proceeded to the third immunization and exhibited a high antibody titer, and subjected to cell fusion in the same manner as above. The screening of hybridomas of 768 specimens was carried out. As a result, two positive samples were detected, each of which was further subjected to the limiting dilution method twice to obtain a monoclonal line (clone #A2-7C6 and #A2-7D11).

In this way, anti-COPB2 monoclonal antibodies of a total of four clones were prepared.

### 3. Preparation of sandwich ELISA

All combinations of the obtained four clones (clone #A3-3G10, #A3-8C9, #A2-7C6, and #A2-7D11) were used as a solid-phase antibody and a labeled antibody in sandwich ELISA and evaluated for their performance. Specifically, each antibody solution prepared at 5 µg/mL with phosphate-buffered saline (PBS) was added at 100 µL/well to a 96-well microplate and reacted overnight. Then, the reaction solution was removed, and the plate was washed once with a washing solution (PBS containing 0.01% Tween 20 and 0.1% ProClin 150). Then, PBS containing 2% BlockAce (Sumitomo Dainippon Pharma Co., Ltd.) and 10% sucrose (FUJIFILM Wako Pure Chemical Corp.) was added at 200 µL/well, and the plate was left at 37°C for 2 hours and thereby blocked to prepare an antibody-immobilized plate.

Meanwhile, all the antibodies were each reacted with a 20-fold molar quantity of Sulfo-NHS-LC-Biotin (Thermo Fisher Scientific Inc.) at room temperature for 2 hours and subjected to gel filtration through Zeba desalting column (Thermo Fisher Scientific Inc.) to prepare labeled antibodies.

The purified COPB2 protein was added at concentrations of 0, 0.1, 1, and 10 ng/mL to the antibody-immobilized plate, stirred, and then reacted at room temperature for 2 hours. Then, the reaction solution was removed, and the plate was washed three times with the washing solution. Then, 1 µg/mL each of the labeled antibodies prepared as described above was added at 100 µL/well, stirred, and then reacted at room temperature for 2 hours. After washing three times, HRP (horseradish peroxidase)-labeled streptavidin (R&D Systems, Inc.) was added at 100 µL/well and reacted at room temperature for 20 minutes. Likewise, after washing three times, a TMB solution (Colorburst Blue, MOSS) was added at 100 µL/well and reacted at room temperature for 20 minutes. The reaction was terminated by the addition of 2 N sulfuric acid thereto at 50 µL/well, and absorbance at 450 nm was measured for each well (Figure 3). As a result, it was confirmed that the strongest signal was detected using clone #A2-7D11 as a solid-phase antibody and clone #A2-7C6 as a labeled antibody among the four anti-COPB2 antibodies.

### 4. Study on Western blot

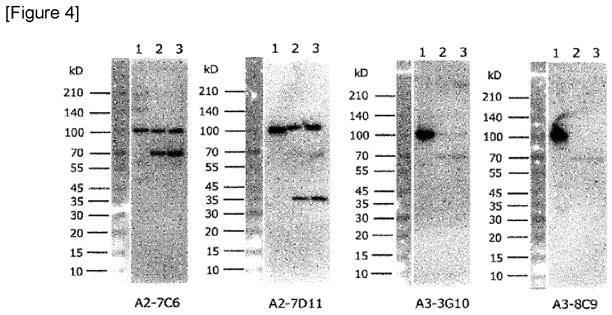

A study was made on whether the antibodies of four clones could detect the COPB2 protein in human cells and mouse cells by Western blot. Specifically, 10 µg of a cell extract prepared from human-derived HEK293 cells with RIPA lysis solution (manufactured by Santa Cruz Biotechnology, Inc.) and 5 or 10 µg of a tissue extract prepared from the mouse liver with T-PER lysis solution (manufactured by Thermo Fisher Scientific Inc.) were each subjected to SDS-PAGE under reducing conditions and then transferred to Immobilon-P membrane (Merck Millipore), which was then blocked in TBS-T (0.05 M Tris-HCl, 0.15 M NaCl, and 0.05% Tween 20, pH 7.6) containing 3% skim milk at room temperature for 1 hour and reacted overnight at 4°C with a solution of each antibody dissolved at a concentration of 1 µg/mL in TBS-T containing 3% skim milk. Each membrane thus reacted was washed three times with TBS-T for 10 minutes, then reacted at 4°C for 2 hours with a solution of HRP-labeled rabbit anti-mouse IgG polyclonal antibody (manufactured by Dako) diluted 1000-fold with TBS-T containing 3% skim milk, and washed three times with TBS-T for 10 minutes. Then, the antibody was detected using Chemi-Lumi One Super (manufactured by Nacalai Tesque, Inc.) (Figure 4). As a result, all the antibodies reacted strongly with human COPB2, and it was suggested that clone #A2-7C6 and #A2-7D11 also cross-reacted with mouse COPB2. Also, clone #A3-3G10 weakly cross-reacted with mouse COPB2, and clone #A3-8C9 had no reaction with mouse COPB2.

### 5. Fluorescent immunohistological staining

### a) Provision

1) In order to enhance the hydrophilicity of a glass cover, the glass cover was coated with polyethyleneimine or poly-L-lysine at room temperature for 1 hour.
2) The glass cover was rinsed with sterile water (1 hr, 3 times).
3) The glass cover was thoroughly dried and then sterilized for at least 4 hours under UV light.
4) Cells are cultured on the glass cover. Alternatively, cells were smeared or fixed with Cytospin(TM) to prepare a sample.
5) The cells were lightly washed with PBS or PBS containing 0.1% Tween 20.

### b) Fixation

1) Incubation was performed at room temperature for 5 minutes in 100% methanol cooled to -20°C.
2) Incubation was performed at room temperature for 10 minutes in PBS (pH 7.4) containing 4% paraformaldehyde.
3) The cells were washed three times with ice-cold PBS.

### c) Antigen retrieval (optional)

1) An antigen retrieval solution (100 mM Tris and 5% [w/v] urea, pH 9.5) is prewarmed to 95°C. A staining jar was filled with the warmed solution and, in this state, dipped in a water bath of 95°C.
2) The glass cover was carefully loaded in the antigen retrieval solution in the staining jar using tweezers having flat tips.
3) Incubation was performed at 95°C for 10 minutes.
4) The glass cover was taken out of the antigen retrieval solution and dipped, with its cell-containing surface facing upward, in a 6-well plate for tissue culture filled with PBS in advance.
5) The sample was washed three times with PBS for 5 minutes.

### d) Permeation treatment

1) The sample was incubated for 10 minutes in PBS containing 0.1 to 0.25% Triton X-100 (or 100 µM digitonin, 0.5% saponin, etc.).
2) Triton X-100 is a surfactant most widely used in permeation treatment but significantly damages cell membranes as compared with digitonin or saponin. Therefore, Triton X-100 needs to be used with attention in the case of also staining a target present on cell membranes. In such a case, a study was desirably made on the concentration of Triton X-100 for each experiment.
3) The cells thus incubated were washed three times with PBS for 5 minutes.

### e) Blocking and immunostaining

1) In order to block the nonspecific binding of an antibody, the sample was incubated at room temperature for 30 minutes in a blocking solution (PBST (PBS + 0.1% Tween 20) containing 1% BSA and 22.52 mg/mL glycine). 1% gelatin, 10% serum (derived from the same animal species as that of a host for a secondary antibody), or the like can also be used instead of the solution described above.
2) The sample was incubated at room temperature for 1 hours or overnight at 4°C with a primary antibody diluted with PBST containing 1% BSA in a wet box.
3) The antibody solution was removed from the sample, and the sample was washed three times with PBS for 5 minutes.
4) The sample was incubated at room temperature for 1 hour with a fluorescently labeled secondary antibody (Thermo, A28175, Goat anti-Mouse IgG (H + L), Superclonal(TM) Recombinant Secondary Antibody, Alexa Fluor(TM) 488) diluted with PBST containing 1% BSA in the dark.
5) The secondary antibody solution was removed from the sample, and the sample was washed three times with PBS for 5 minutes in the dark, if possible.

### f) Nuclear counterstaining

1) 0.1 to 1 µg/mL Hoechst33342 nuclear staining agent (Thermo, H3570) or DAPI was added to the sample and incubated for 1 minute.
2) The sample was washed with PBS.

### g) Mounting

1) One drop of a mountant was added to the sample, which was then sealed with the glass cover.
2) In order to prevent the sample from moving, the glass cover was sealed with a transparent manicure solution.
3) The resultant was preserved at 4°C or -20°C in the dark.

### h) Microscopic observation

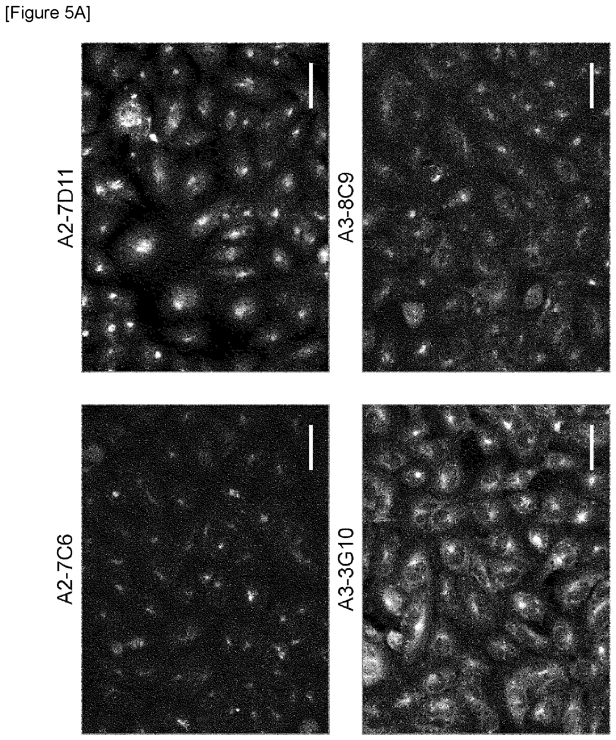

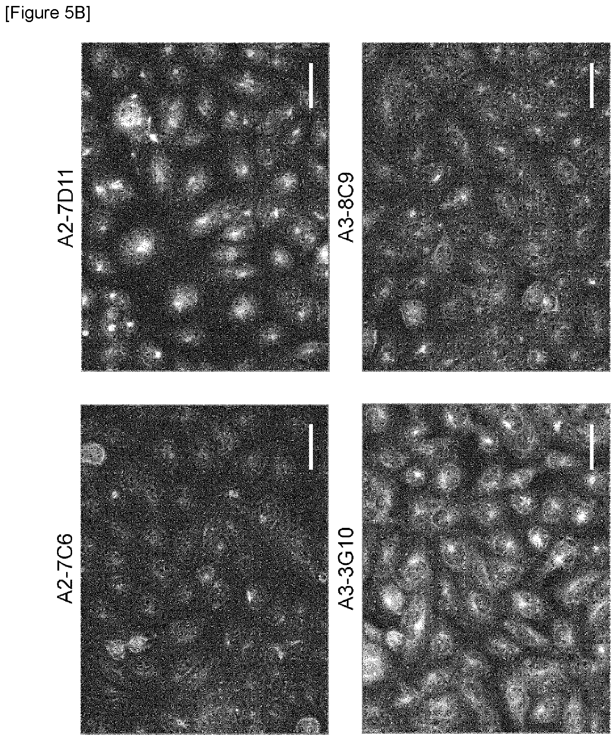

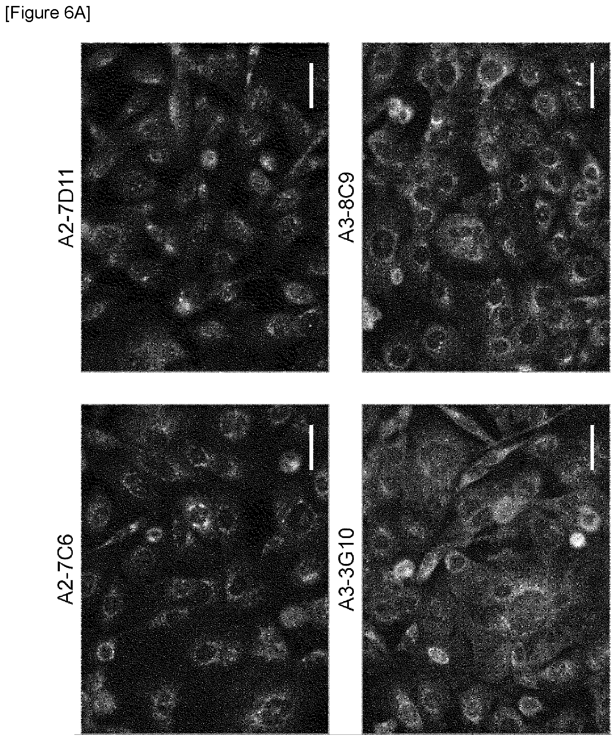

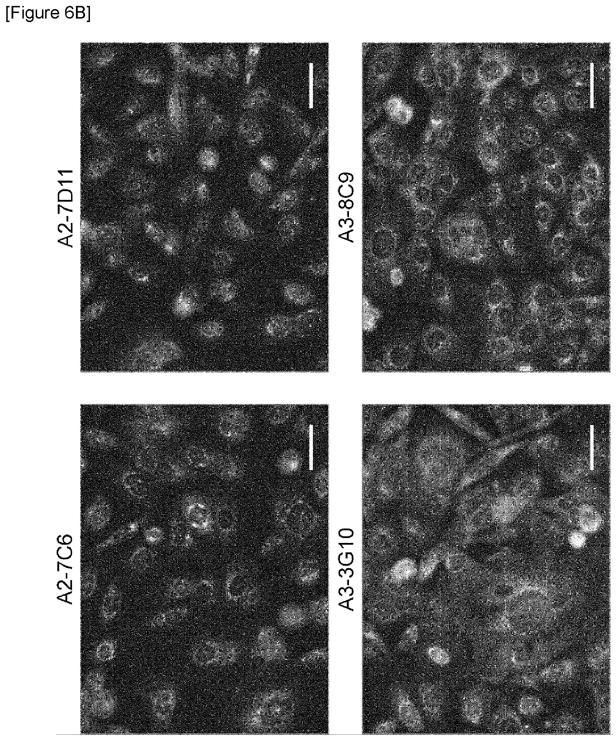

Observation was performed under a fluorescence microscope (Keyence BZ-X810), and images were recorded (Figures 5 and 6).

### 6. Structural analysis of anti-COPB2 antibody

The heavy chain and light chain variable regions of the anti-COPB2 monoclonal antibodies A2-7C6, A2-7D11, and A3-8C9 were structurally analyzed with Bioinformatic Analysis System of Repertoire Genesis Inc.

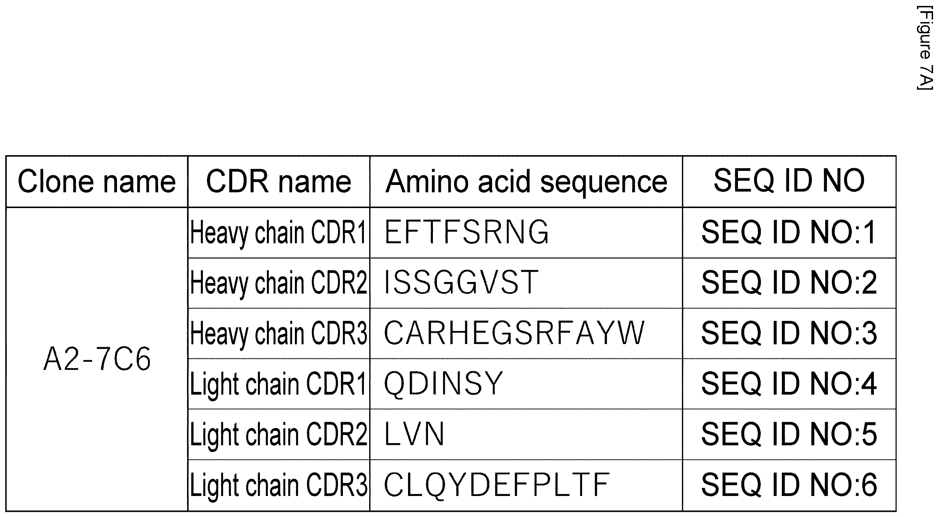

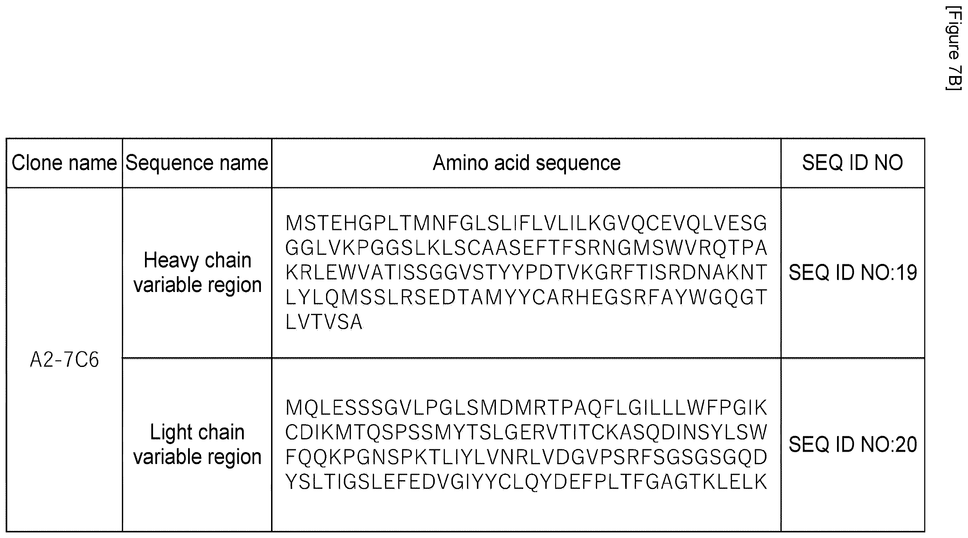

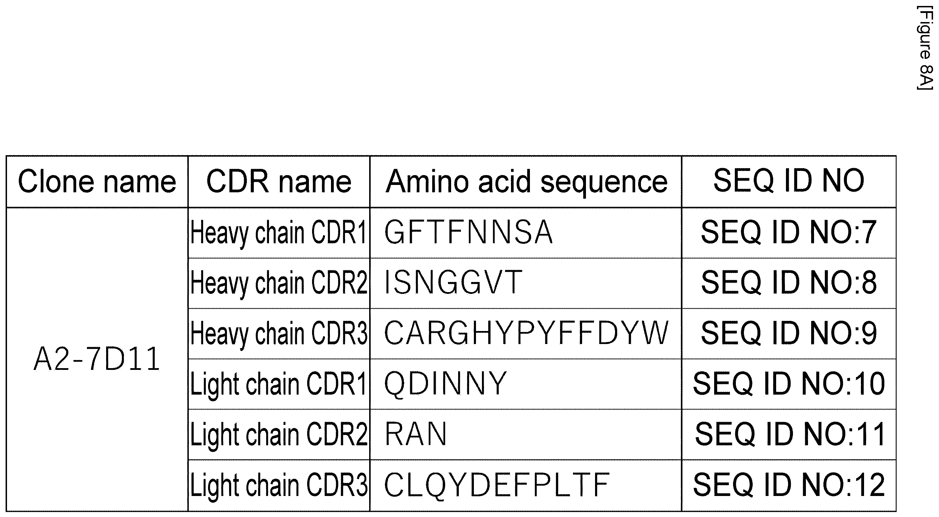

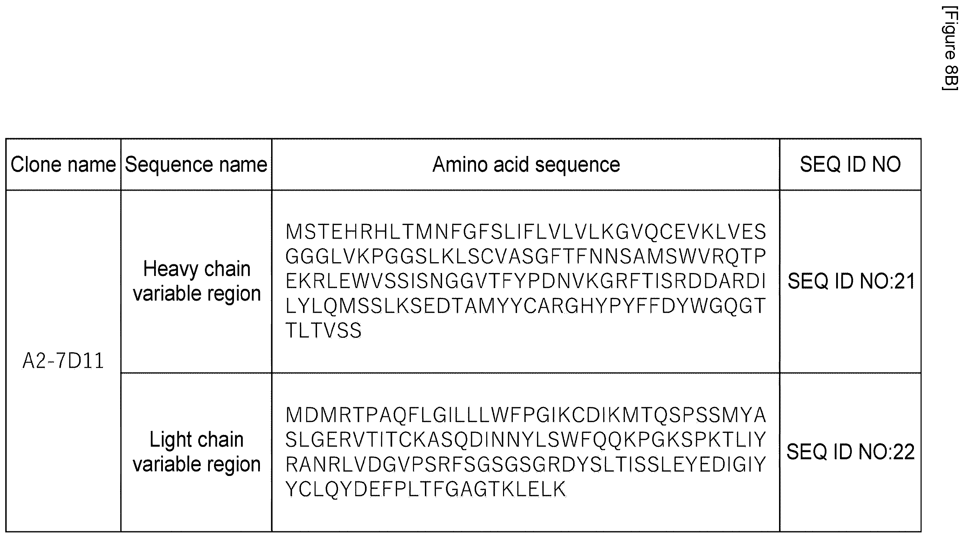

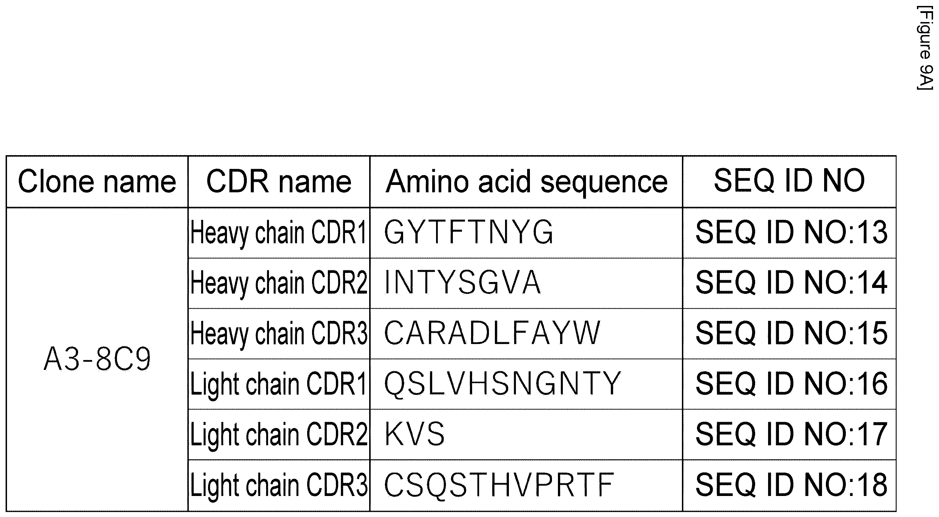

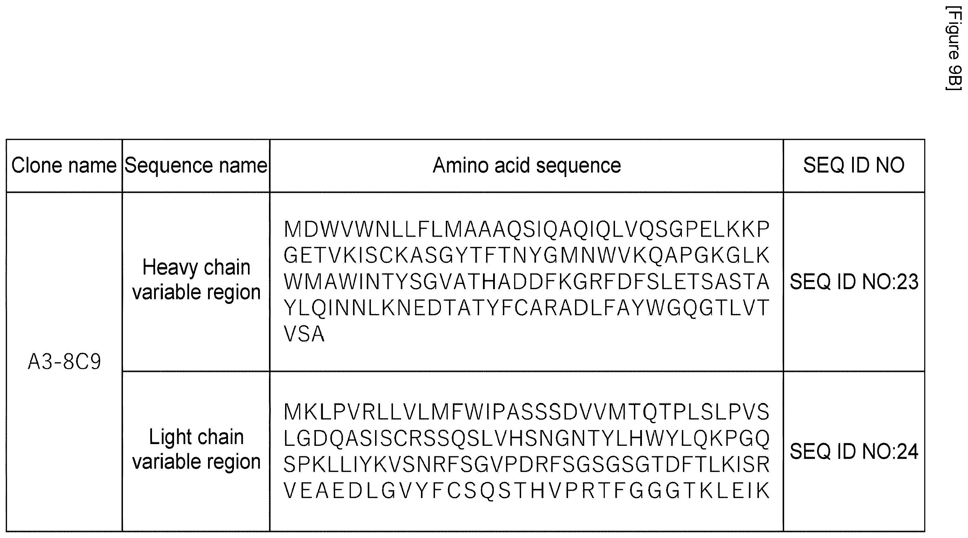

Figures 7 to 9 show the amino acid sequences of heavy chain CDR1 to CDR3 and light chain CDR1 to CDR3 and the amino acid sequences of the heavy chain and light chain variable regions in the anti-COPB2 monoclonal antibodies A2-7C6, A2-7D11, and A3-8C9.

## Claims

1. An antibody against coatomer protein complex subunit beta 2 (CO PB2) or an antigen binding fragment thereof, comprising any of the following CDR sets (a) to (c):
(a)
heavy chain CDR1 consisting of the amino acid sequence represented by SEQ ID NO: 1,
heavy chain CDR2 consisting of the amino acid sequence represented by SEQ ID NO: 2,
heavy chain CDR3 consisting of the amino acid sequence represented by SEQ ID NO: 3,
light chain CDR1 consisting of the amino acid sequence represented by SEQ ID NO: 4,
light chain CDR2 consisting of the amino acid sequence represented by SEQ ID NO: 5, and
light chain CDR3 consisting of the amino acid sequence represented by SEQ ID NO: 6,
(b)
heavy chain CDR1 consisting of the amino acid sequence represented by SEQ ID NO: 7,
heavy chain CDR2 consisting of the amino acid sequence represented by SEQ ID NO: 8,
heavy chain CDR3 consisting of the amino acid sequence represented by SEQ ID NO: 9,
light chain CDR1 consisting of the amino acid sequence represented by SEQ ID NO: 10,
light chain CDR2 consisting of the amino acid sequence represented by SEQ ID NO: 11, and
light chain CDR3 consisting of the amino acid sequence represented by SEQ ID NO: 12,
(c)
heavy chain CDR1 consisting of the amino acid sequence represented by SEQ ID NO: 13,
heavy chain CDR2 consisting of the amino acid sequence represented by SEQ ID NO: 14,
heavy chain CDR3 consisting of the amino acid sequence represented by SEQ ID NO: 15,
light chain CDR1 consisting of the amino acid sequence represented by SEQ ID NO: 16,
light chain CDR2 consisting of the amino acid sequence represented by SEQ ID NO: 17, and
light chain CDR3 consisting of the amino acid sequence represented by SEQ ID NO: 18.

2. The antibody against coatomer protein complex subunit beta 2 (COPB2) or the antigen binding fragment thereof according to claim 1, comprising any of the following heavy chain variable regions and/or light chain variable regions (d) to (f):
(d)
a heavy chain variable region comprising the amino acid sequence represented by SEQ ID NO: 19;
a heavy chain variable region comprising an amino acid sequence differing by having addition, substitution, or deletion of one to several amino acids from the amino acid sequence represented by SEQ ID NO: 19; or
a heavy chain variable region comprising an amino acid sequence having 80% or higher identity to the amino acid sequence represented by SEQ ID NO: 19, and/or
a light chain variable region comprising the amino acid sequence represented by SEQ ID NO: 20;
a light chain variable region comprising an amino acid sequence differing by having addition, substitution, or deletion of one to several amino acids from the amino acid sequence represented by SEQ ID NO: 20; or
a light chain variable region comprising an amino acid sequence having 80% or higher identity to the amino acid sequence represented by SEQ ID NO: 20,
(e)
a heavy chain variable region comprising the amino acid sequence represented by SEQ ID NO: 21;
a heavy chain variable region comprising an amino acid sequence differing by having addition, substitution, or deletion of one to several amino acids from the amino acid sequence represented by SEQ ID NO: 21; or
a heavy chain variable region comprising an amino acid sequence having 80% or higher identity to the amino acid sequence represented by SEQ ID NO: 21, and/or
a light chain variable region comprising the amino acid sequence represented by SEQ ID NO: 22;
a light chain variable region comprising an amino acid sequence differing by having addition, substitution, or deletion of one to several amino acids from the amino acid sequence represented by SEQ ID NO: 22; or
a light chain variable region comprising an amino acid sequence having 80% or higher identity to the amino acid sequence represented by SEQ ID NO: 22, and (f)
a heavy chain variable region comprising the amino acid sequence represented by SEQ ID NO: 23;
a heavy chain variable region comprising an amino acid sequence differing by having addition, substitution, or deletion of one to several amino acids from the amino acid sequence represented by SEQ ID NO: 23; or
a heavy chain variable region comprising an amino acid sequence having 80% or higher identity to the amino acid sequence represented by SEQ ID NO: 23, and/or
a light chain variable region comprising the amino acid sequence represented by SEQ ID NO: 24;
a light chain variable region comprising an amino acid sequence differing by having addition, substitution, or deletion of one to several amino acids from the amino acid sequence represented by SEQ ID NO: 24; or
a light chain variable region comprising an amino acid sequence having 80% or higher identity to the amino acid sequence represented by SEQ ID NO: 24.

3. A nucleic acid encoding the antibody or the antigen binding fragment thereof according to claim 1 or 2.

4. An expression vector comprising the nucleic acid according to claim 3.

5. A transformant comprising the expression vector according to claim 4.

6. A kit for detecting COPB2 protein in a sample, comprising the antibody or the antigen binding fragment thereof according to claim 1 or 2.

7. The kit according to claim 6, wherein the detection is based on an immunohistochemical method (IHC), an immunofluorescent method (IF), flow cytometry, enzyme-linked immunosorbent assay (ELISA), or immunoblot.
